# EUROPEAN PATENT APPLICATION

(11) **EP 4 678 739 A1**
(43) Date of publication of application: **14.01.2026**
(21) Application number: 24382739.1
(22) Date of filing: 10.07.2024
(51) Int. Cl.: C12N 5/0793

(54) **IN VITRO METHOD FOR OBTAINING CORTICAL BRAIN ORGANOIDS, CORTICAL BRAIN ORGANOIDS AND USES THEREOF**

(71) Applicant: Fundación Pública Andaluza Progreso y Salud, 41092 Sevilla (ES); Administración General de la Comunidad Autónoma de Euskadi, 01010 Vitoria-Gasteiz (Araba/Alava) (ES); Universidad de Sevilla, 41013 Sevilla (ES)
(72) Inventor: GARCÍA DELGADO, Ana Belén, 41013 Sevilla (ES); FERNÁNDEZ MUÑOZ, Beatriz, 41013 Sevilla (ES); SÁNCHEZ PERNAUTE, Rosario, 48903 Barakaldo (ES)
(74) Representative: Hoffmann Eitle

(57) **Abstract**

The present invention refers to the biomedical field. Particularly, the present invention refers to an *in vitro* method for obtaining a cortical brain organoid, a cortical brain organoid obtained or obtainable by said method and its application as a medicament, preferably in a method of treating a brain disorder, condition, or disease.

## Description

### FIELD OF THE INVENTION

The present invention refers to the biomedical field. Particularly, the present invention refers to an *in vitro* method for obtaining a cortical brain organoid, a cortical brain organoid obtained or obtainable by said method and its application as a medicament, preferably in a method of treating a brain disorder, condition, or disease.

### STATE OF THE ART

The cortical region of the brain, known as the cerebral cortex, is a crucial part of the brain responsible for many high-order functions, including sensory perception, cognition, motor control, and language. It is composed of several layers of neurons and plays a key role in processing information and executing complex tasks. Due to its complexity and significance in brain function, the cortical region is often affected by a variety of neurological and psychiatric disorders, such as Alzheimer's disease, epilepsy, autism, and schizophrenia. These conditions can lead to devastating impacts on individuals' cognitive abilities, emotional regulation, and overall quality of life.

Currently, modeling diseases that affect the cortical region of the brain poses significant challenges due to the lack of accurate and replicable *in vitro* systems. Traditional animal models and simple cell cultures often fail to capture the intricate architecture and functionality of the human cerebral cortex. This limitation hampers our ability to investigate the molecular mechanisms underlying these diseases effectively. Developing an *in vitro* model of the cortical region, such as cortical brain organoids, would be a groundbreaking advancement. Cortical organoids would offer a valuable platform for studying disease pathogenesis, identifying potential drug targets, and screening therapeutic compounds. Furthermore, some of these models could be used in regenerative medicine approaches, potentially leading to novel treatments that can restore or replace damaged brain tissue in affected individuals. This would mark a significant step forward in our ability to understand, diagnose, and treat cortical brain diseases.

Thus, there is an unmet medical need of developing *in vitro* models for the cortical region of the brain. The present invention is focused on solving this problem, and an *in vitro* method for obtaining a cortical brain organoid that could be applied to the treatment of a brain disorder, condition, or disease, is herein provided.

### DESCRIPTION OF THE INVENTION

### Brief description of the invention

The present invention refers to an *in vitro* method for obtaining a cortical brain organoid, a cortical brain organoid obtained or obtainable by said method and its application as a medicament, preferably in a method of treating a brain disorder, condition, or disease.

The inventors of the present invention have developed a protocol for the generation of cortical brain organoids with improved cortical properties.

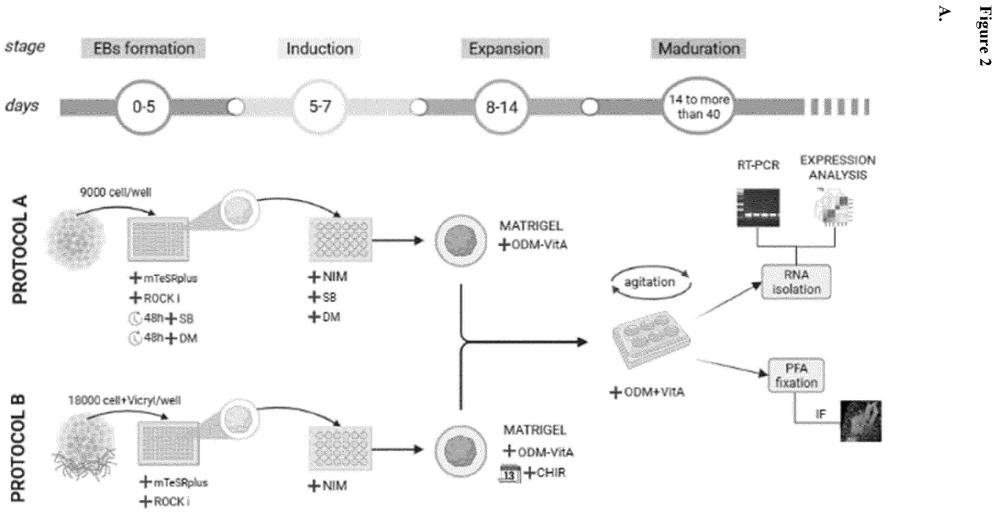

First, they compared cortical organoids generated from a protocol A, characterized by the addition of dual-SMAD inhibitory molecules of BMP and TFG-β pathways (SB431542 and Dorsomorphin), with an alternative protocol B in which these inhibitors were not added to the medium **(****Figure 2A****).**

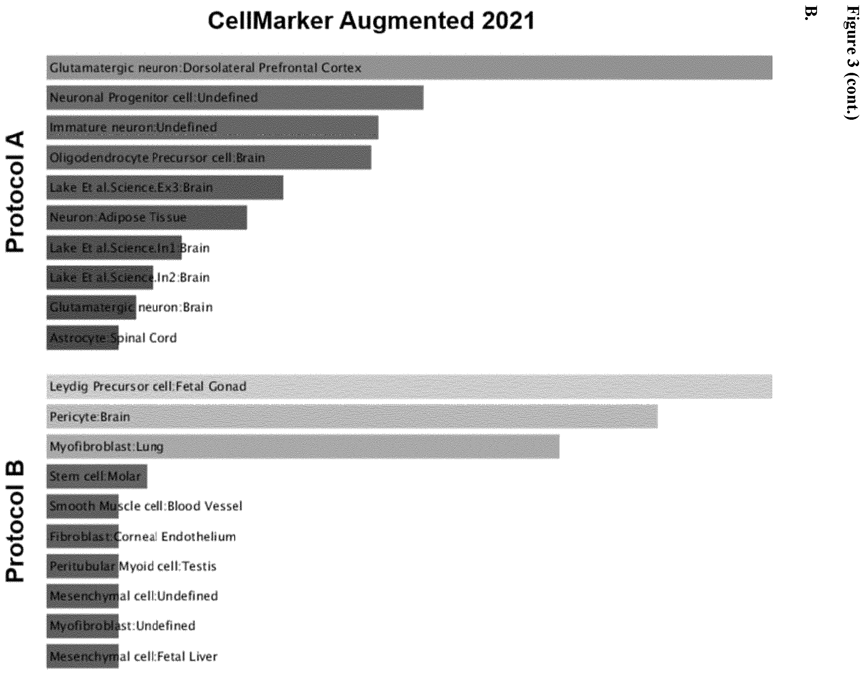

A transcriptomic analysis revealed that organoids derived from protocol A were enriched in functional terms related to neuronal differentiation, indicating a greater representation of glutamatergic neurons from the dorsolateral prefrontal cortex, while those derived from protocol B were enriched in functional terms related to non-neuronal fates, mostly related to cardiac tissue **(****Figure 3B****).** This analysis shows that the dual-SMAD inhibitory molecules SB431542 and Dorsomorphin added in protocol A, have a positive effect on the organoids enriching them in neuroectoderm tissue, while organoids generated without these molecules in protocol B are contaminated with other non-ectodermal layers, developing other tissues like cardiac.

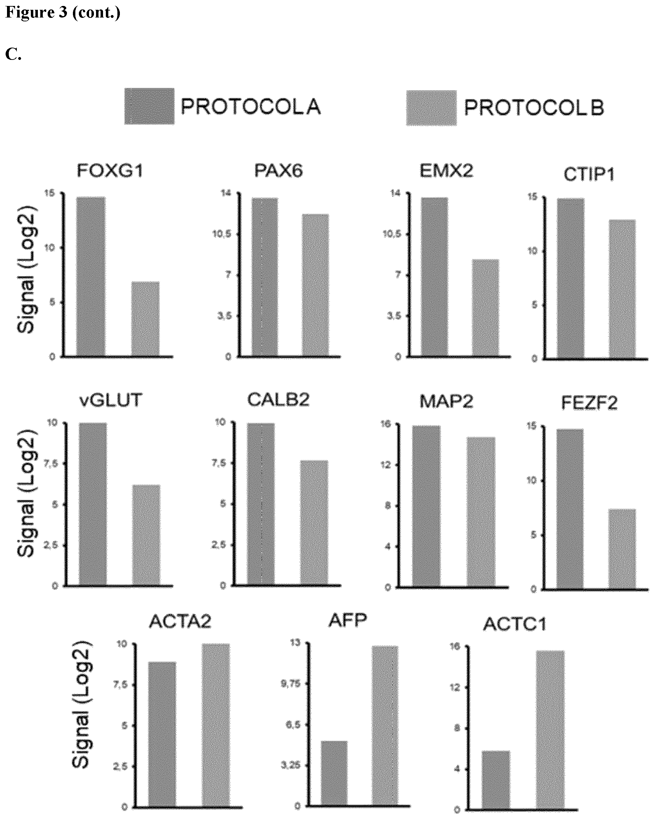

Moreover, organoids derived from protocol A displayed an increased expression of cortical genes, such as FOXG1, EMX2, PAX6, CTIP1, FEZF2, MAP2, CALB2 and vGLUT **(****Figure 3C****).** Overall, these results suggest that those organoids generated from protocol A display a more cortical identity. By contrast, organoids generated following protocol B showed a higher expression of mesoderm and endoderm markers.

Together, these results make plausible the use of protocol A in the generation of cortical brain organoids (CBOs).

The inventors then explored the possibility of further enhancing the cortical identity of the organoids generated using protocol A by embedding the organoids in decellularized porcine cortical ECM (cECM) instead of Matrigel. In this case, they compared organoids embedded in Matrigel and organoids embedded in porcine cECM (both generated using protocol A).

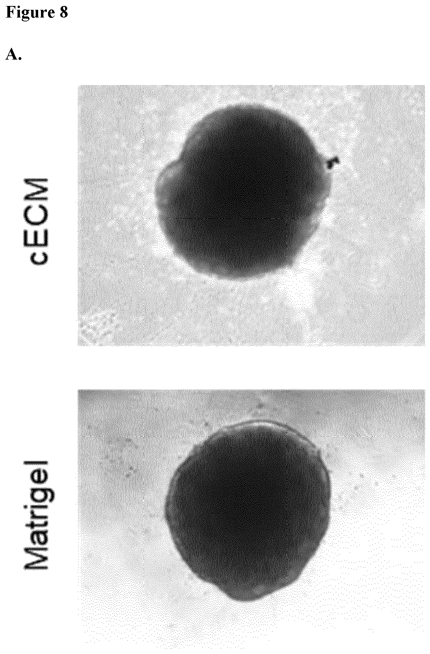

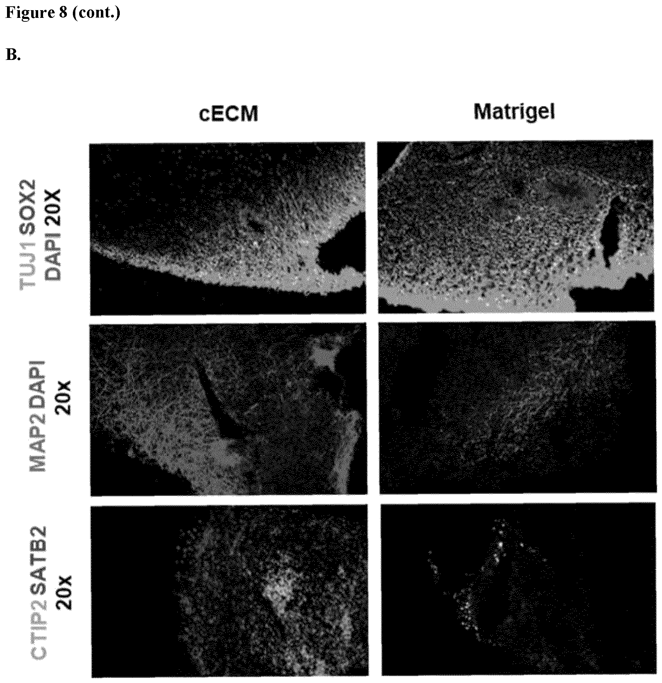

While no major differences were observed under phase contrast microscopy across groups, organoids embedded in cECM showed higher neuronal projections to the outer zone of the CBOs **(****Figure 8A****).** Organoids embedded in decellularized porcine cECM displayed a much better cortical organization than the ones embedded in Matrigel, showed by their expression of CTIP2 and SATB2 **(****Figure 8B****).** These results show that the use of cECM has a positive effect on the organoid generation process, resulting in organoids that display a more developed cortical structure.

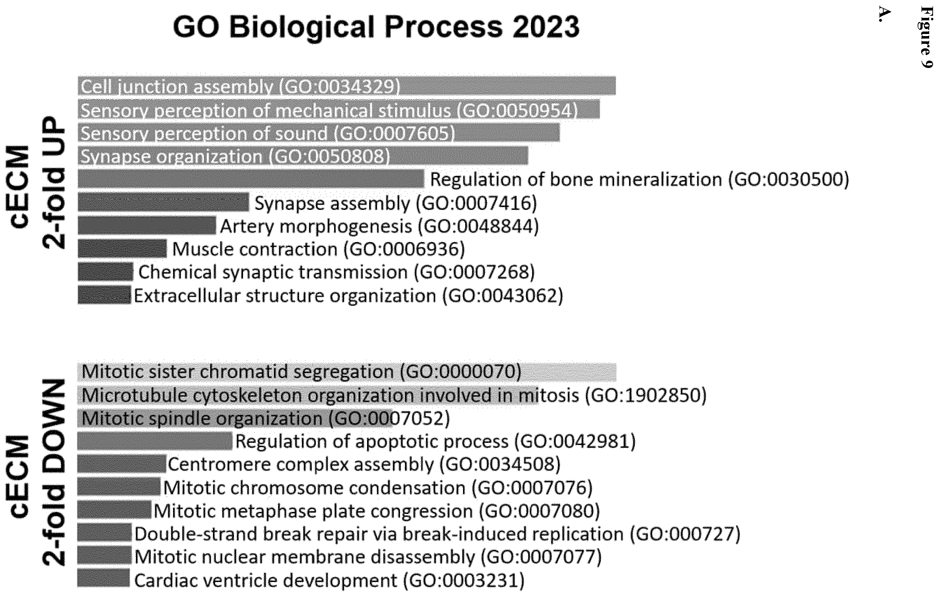

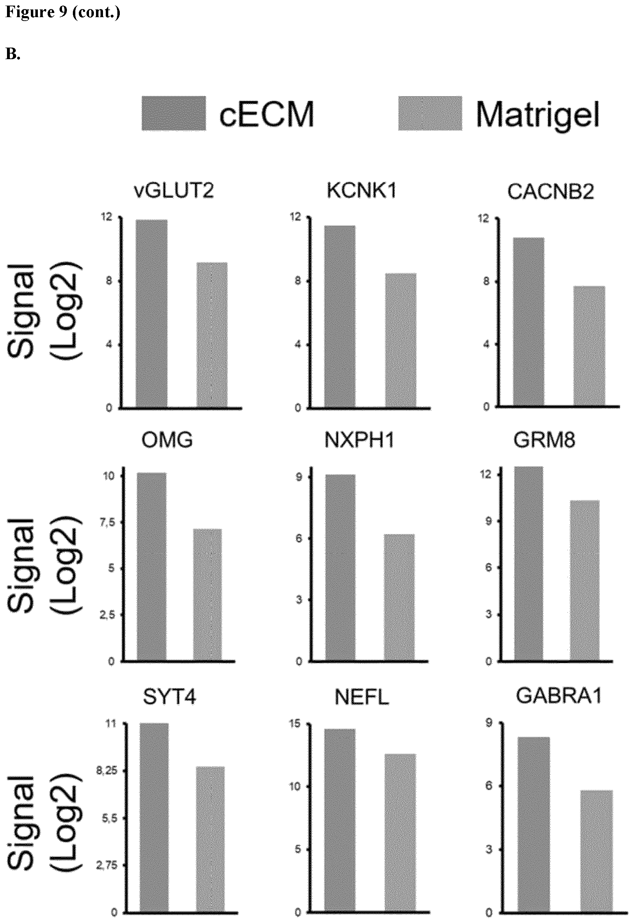

Moreover, a transcriptomic analysis revealed an enrichment of functional terms related to synaptic processes in organoids embedded in cECM compared to those embedded in Matrigel **(****Figure 9A****),** as well as an upregulation of genes related to synaptic processes **(****Fig. 9B****).** suggesting that embedding organoids in cECM has a positive effect in neuronal maturation within the organoids.

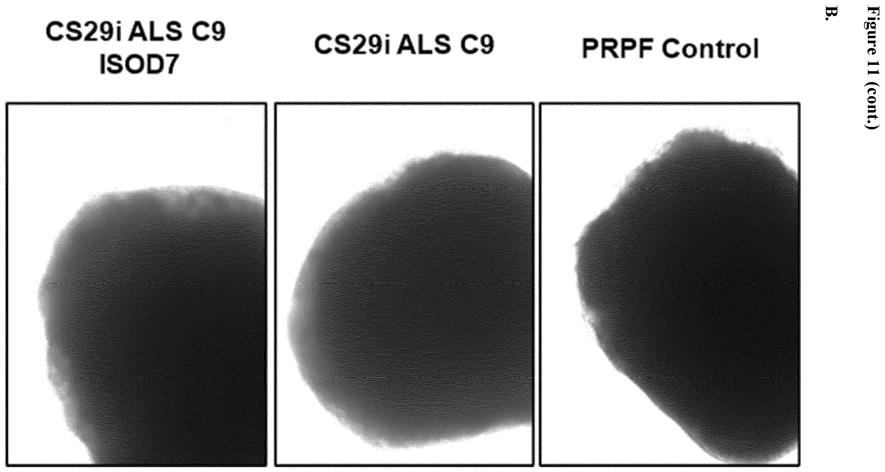

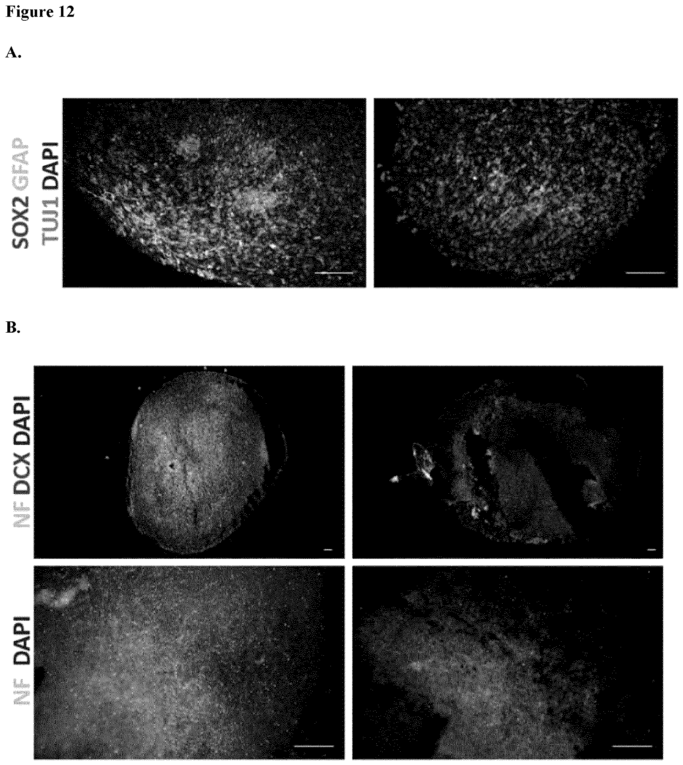

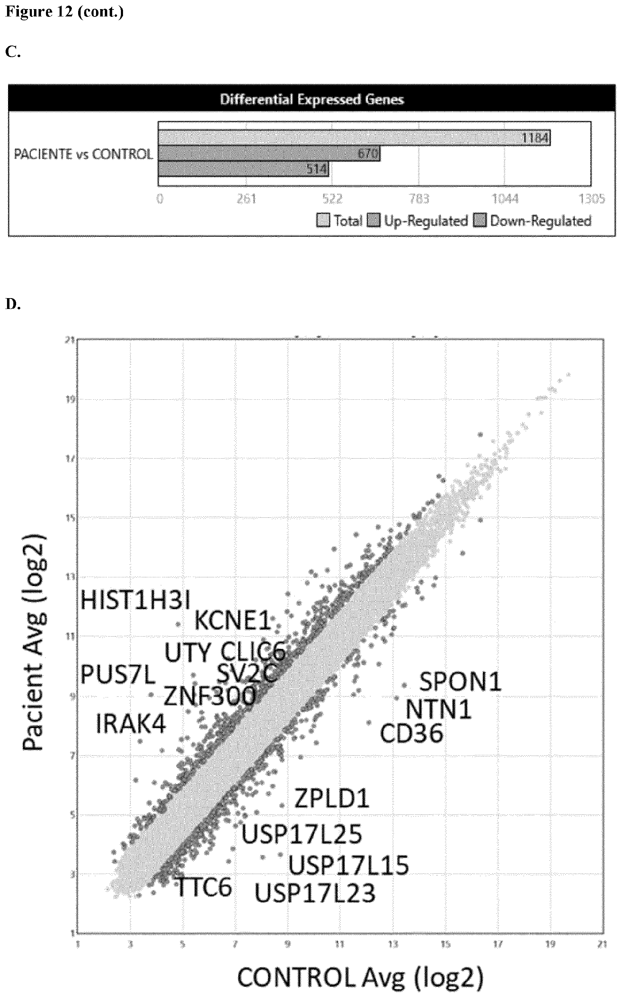

Finally, the inventors used two human iPSCs (hiPSCs) from amiotrophic lateral sclerosis (ALS) patients to generate a CBO model to study the disease. For this, they used protocol A with the optimization of embedding the organoids in porcine cECM. They also included the hiPSCs line PRPF control as a positive control. All hiPSCs lines successfully generated brain-like organoids. Organoids generated from hiPSCs derived from the ALS patient showed a similar structure as those generated from the positive control hiPSCs line PRPF control **(****Figure 11B****).** After 2 months of maturation, ALS organoids display alterations in neurons such as degradation of neurofilaments (NF), detected by low intracellular expression of NF proteins **(****Figure 12B****).** NF levels increase in biological fluids (blood, cerebrospinal fluid) of ALS patients probably due to axon damage and NF degradation and measurement of NF levels is routinely used as a diagnosis and prognostic test in clinics. Furthermore, ALS organoids show a deregulation of pathways previously related to ALS physiopathology such as the ubiquitin-proteasome pathway **(****Figure 12C-G**). Deregulation of the ubiquitin-proteasome pathway is mainly observed at the level of de-ubiquitinating enzymes, especially ubiquitin-specific proteases (USP) such us USP17 family, that is strongly downregulated **(****Figure 12D-E**).

Together, these results indicate that protocol A can be used as a protocol for the generation of cortical brain organoids with improved cortical properties, that the cortical identity of these organoids can be further improved by embedding the organoids with cECM, and that this protocol can be successfully used for the generation of CBOs that may serve to model conditions that affect the cortical region of the brain, such as ALS. It is important to note that the use of a regionalized matrix (i.e. the use of an ECM obtained from the cortical region of the brain rather than from any other region) is one of the features that differentiates this protocol from other existing protocols for the generation of CBOs.

### Detailed description of the embodiments

In view of the above, a first aspect of the invention refers to an *in vitro* method for obtaining a cortical brain organoid, which comprises:
a) culturing one or more pluripotent stem cells (PSCs), in a maintenance medium for PSCs,
b) culturing the cells resulting from step a) in a neural induction medium, and
c) culturing the embryonic bodies resulting from step b) in contact with an extracellular matrix in the presence of a cortical differentiation medium comprising B27 supplement and/or N2 supplement, wherein the extracellular matrix is characterized in that it is a decellularized brain cortical extracellular matrix.

Regarding step a) of the first aspect of the invention:
- In a preferred embodiment, the one or more pluripotent stem cells (PSCs) are selected from: embryonic stem cells (ESCs) derived from a blastocyst stage embryo, induced pluripotent stem cells (iPSCs), parthenogenetic PSCs and PSCs generated by nuclear transfer.
- In a preferred embodiment the maintenance medium for PSCs is a feeder-free maintenance medium for PSCs.
- In a preferred embodiment, the maintenance medium for PSCs of a) comprises at least 90 ng/ml FGF-2, a ROCK inhibitor, and/or one or more inhibitors of the SMAD pathway.
   ∘ Preferably, the concentration of FGF-2 is between 90 and 100 ng/ml.
   ∘ Preferably, the ROCK inhibitor is selected from the list consisting of: Y-27632, Fasudil (HA-1077), Ripasudil (K-115), H-1152, GSK269962A, SR3677, Thiazovivin, RKI-1447, Chroman 1 and Blebbistatin, preferably wherein the ROCK inhibitor is Y-27632.
   ∘ Preferably, the one or more inhibitors of the SMAD pathway are selected from the list consisting of: SB431542, LY2157299 (Galunisertib), A-83-01, SD-208, RepSox (E-616452), LY2109761, SB525334, LY364947, Pirfenidone, SB505124, GW788388, Vactosertib (TEW-7197), ITD-1, Halofuginone, Sulfasalazine, LY 3200882, TP0427736 HCl, TGFβRI-IN-3, PD 169316, 3,3-Dimethyl-1-butanol, Lycopus Extract, AUDA, BIBF-0775, Ginsenoside Rh4, R-268712 and/or TA-02, Dorsomorphin, Dorsomorphin 2HCl, LDN-193189, LDN-193189 HCl, DMH1, K02288, LDN-212854, MI,347, LDN-214117, SIS3 and/or SIS3 HCl. Preferably, the one or more inhibitors of the SMAD pathway are SB431542 and Dorsomorphin.

Regarding step b) of the first aspect of the invention:
- In a preferred embodiment, the neural induction medium of b) comprises N2 supplement and/or one or more inhibitors of the SMAD pathway, wherein the N2 supplement is preferably at a concentration of at least 1%.
   ∘ The one or more inhibitors of the SMAD pathway are the same as for step a). Thus, all the embodiments mentioned in this regard for step a) apply herein.

Regarding step c) of the first aspect of the invention:
- In a preferred embodiment the decellularized brain cortical extracellular matrix is a porcine decellularized brain cortical extracellular matrix.
- In a preferred embodiment, the decellularized brain cECM is a decellularized brain cECM obtained from the motor cortex. It is important to note that the examples provided in this patent application were performed using brain cECM obtained from the motor cortex because the intention was to model ALS, wherein the primary motor cortex is predominantly affected. However, if the intention were to model a disease that primarily affects another region of the cortex, it would be more suitable to use brain cECM obtained from that specific region of the cortex.
- In a preferred embodiment, step c) involves culturing the embryonic bodies resulting from step b) in contact with an extracellular matrix in the presence of a first cortical differentiation medium comprising B27 without vitamin A supplement and/or N2 supplement, and then a second cortical differentiation medium comprising B27 with vitamin A supplement and/or N2 supplement, wherein the extracellular matrix is characterized in that it is a decellularized brain cortical extracellular matrix.

In a preferred embodiment:
i) the maintenance medium for PSCs of a) comprises at least 90 ng/ml FGF-2, a ROCK inhibitor, and one or more inhibitors of the SMAD pathway,
ii) wherein the ROCK inhibitor is Y-27632,
iii) the neural induction medium of b) comprises N2 supplement at a concentration of at least 1% and one or more inhibitors of the SMAD pathway, and
iv) wherein the one or more inhibitors of the SMAD pathway are SB431542 and Dorsomorphin.

A second aspect of the invention refers to a cortical brain organoid obtained or obtainable by the method of the first aspect of the invention. Importantly, the methodology of the first embodiment of the invention confers the organoids some properties that are not present in organoids generated by other existing methods, namely a greater neuronal maturity and a better specification towards the cortical program.

A third aspect of the invention refers to the cortical brain organoid of the second aspect of the invention, for use as a medicament.

In a preferred embodiment, the cortical brain organoid is for use in a method of treating a brain disorder, condition, or disease.

In a preferred embodiment, the brain disorder, condition, or disease is a brain disorder, condition or disease that affects the cortical region of the brain. Preferably, the brain disorder, condition, or disease is selected from the list consisting of: amyotrophic lateral sclerosis (ALS), Alzheimer's disease, Parkinson's disease, epilepsy, dementia, encephalitis, cerebral tumor, cerebrovascular accident, Huntington's disease, Creutzfeldt-Jakob disease, Wilson's disease, Rasmussen's encephalitis, aphasia, frontotemporal dementia (FTD), cortical dysplasia, stroke, cerebral palsy, progressive supranuclear palsy, Lewy body dementia (LBD), cortical visual impairment (CVI), schizophrenia, autism spectrum disorder (ASD) and posterior cortical atrophy (PCA). More preferably, the brain disorder, condition, or disease is amyotrophic lateral sclerosis (ALS).

A fourth aspect of the invention refers to the use of the cortical brain organoid of the second aspect of the invention for screening candidate compounds suitable for use in a method of treating a brain disorder, condition, or disease.

The brain disorder, condition, or disease is the same as for the third aspect. Thus, all the embodiments in this regard for the third aspect apply herein.

A fifth aspect of the invention refers to a method of treating a brain disorder, condition, or disease in a subject comprising administering the organoids of the second aspect of the invention.

The brain disorder, condition, or disease is the same as for the third aspect. Thus, all the embodiments in this regard for the third aspect apply herein.

A sixth aspect of the invention refers to the use of a maintenance medium for PSCs as defined in the first aspect of the invention, a neural induction medium as defined in the first aspect of the invention, a cortical differentiation medium as defined in the first aspect of the invention, or a decellularized brain cortical extracellular matrix as defined in the first aspect of the invention, in a method for obtaining a cortical brain organoid.

A seventh aspect refers to the use of a neural induction medium as defined in the first aspect of the invention in a method for the generation of embryonic bodies.

### General considerations

Each embodiment disclosed herein is contemplated as being applicable to each of the other disclosed embodiments. Thus, all combinations of the various elements described herein are within the scope of the invention.

Unless otherwise stated, the terms used in the present application have the meanings indicated herein. Terms that are not defined herein should be given the meanings that would be given to them by a person skilled in the art in the context of this disclosure.

In the context of the present invention the following terms are defined:
- The term "comprising" means including, but it is not limited to, whatever follows the word "comprising". Thus, use of the term "comprising" indicates that the listed elements are required or mandatory, but that other elements are optional and may or may not be present.
- The term "consisting of" means including, and it is limited to, whatever follows the phrase "consisting of". Thus, the phrase "consisting of" indicates that the listed elements are required or mandatory, and that no other elements may be present.
- The term "organoid" refers to a miniaturized and simplified version of an organ produced *in vitro* in three dimensions that shows realistic micro-anatomy. These structures are derived from stem cells, such as pluripotent stem cells (PSCs) or organ-specific adult stem cells, and they self-organize through cell sorting and spatially restricted lineage commitment to mimic the architecture and functionality of real organs. Organoids can replicate many of the key aspects of the organ they model, including specific cell types, functions, and even some disease states, making them valuable tools for studying development, disease mechanisms, and potential treatments in a controlled laboratory environment.
- The term "cortical brain organoid" refers to is a specialized type of organoid that mimics the structure and function of the cerebral cortex, the outermost layer of the brain. These organoids are derived from stem cells and cultured *in vitro* under conditions that promote the development of cortical tissue. Cortical brain organoids exhibit several key features of the human cortex, including the presence of multiple neuronal cell types, the formation of layered structures, and the ability to generate electrical activity similar to that of a developing brain. These organoids serve as valuable models for studying the complex processes of brain development, investigating the molecular and cellular mechanisms underlying cortical brain disorders, and testing potential therapeutic interventions. They offer a unique platform for researchers to explore the pathogenesis of diseases that affect the cortical region, in a controlled and replicable environment.
- The term "pluripotent stem cells (PSCs)" refers to a type of stem cell capable of differentiating into any cell type of the three germ layers: ectoderm, mesoderm, and endoderm. They can give rise to nearly all cell types in the body, including neurons, muscle cells, and blood cells. PSCs have two main sources: embryonic stem cells (ESCs) and induced pluripotent stem cells (iPSCs).
- The term "embryonic stem cells (ESCs) derived from a blastocyst stage embryo" refers to PSCs that originate from the inner cell mass of a developing embryo.
- The term "induced pluripotent stem cells (iPSCs)" refers to cells that have been reprogrammed from adult somatic cells through specific transcription factors. They share similar properties with ESCs and are valuable for research and therapeutic applications because they can be derived from a patient's own cells, reducing the risk of immune rejection.
- The term "parthenogenetic PSCs" refer to a type of stem cell derived from unfertilized eggs (oocytes) through a process called parthenogenesis.
- The term "PSCs generated by nuclear transfer" refers to a type of stem cell derived from reprogrammed somatic cells. Nuclear transfer involves transferring the nucleus of a somatic cell, such as a skin cell, into an enucleated egg cell (oocyte). The egg cell's cytoplasm contains factors that reprogram the somatic cell nucleus back to a pluripotent state, similar to the process of fertilization.
- The term "maintenance medium for PSCs" refers to a specially formulated culture medium designed to support the growth and proliferation of PSCs while preserving their pluripotent state. The maintenance medium provides the necessary nutrients, growth factors, and signalling molecules that prevent differentiation and promote self-renewal. Key components typically found in a maintenance medium for PSCs include:
   ∘ Basal medium: Provides essential nutrients such as amino acids, vitamins, and minerals.
   ∘ Serum or serum replacements: Supplies additional growth factors and proteins. Many PSC maintenance media use serum-free formulations with defined serum replacements to reduce variability and improve consistency.
   ∘ Growth factors: Essential for maintaining pluripotency, such as basic fibroblast growth factor (bFGF or FGF2), and sometimes other factors like transforming growth factor-beta (TGF-β) or leukemia inhibitory factor (LIF).
   ∘ Small molecules and inhibitors: Compounds that enhance the self-renewal and pluripotency of PSCs by modulating specific signalling pathways. Examples include MEK inhibitors, GSK3 inhibitors, and TGF-β pathway inhibitors.
   ∘ Other supplements: Such as non-essential amino acids, antioxidants, and antibiotics/antimycotics to support cell health and prevent contamination.
   The maintenance medium is carefully optimized to create an environment that mimics the natural conditions in which PSCs thrive, ensuring they remain undifferentiated and capable of generating various cell types for extended periods in culture.
- The term "feeder-free maintenance medium for PSCs" refers to a specialized culture medium that supports the growth and maintenance of PSCs without the need for a feeder cell layer. Feeder cells, typically mouse embryonic fibroblasts (MEFs), are traditionally used to provide the necessary growth factors and extracellular matrix components that help maintain PSC pluripotency. However, feeder-free systems have been developed to eliminate the need for these feeder cells, reducing variability and the risk of contamination. Key components of a feeder-free maintenance medium for PSCs include:
   ∘ Basal medium: Provides essential nutrients, such as amino acids, vitamins, and minerals.
   ∘ Serum-free formulations: Typically include defined serum replacements or chemically defined supplements that provide the necessary growth factors and proteins without the variability associated with serum.
   ∘ Growth factors: Essential for maintaining pluripotency, such as basic fibroblast growth factor (bFGF or FGF2), and sometimes other factors like transforming growth factor-beta (TGF-β) or Activin A.
   ∘ Small molecules and inhibitors: Compounds that enhance the self-renewal and pluripotency of PSCs by modulating specific signaling pathways. Examples include MEK inhibitors, GSK3 inhibitors, and TGF-β pathway inhibitors.
   ∘ Extracellular matrix (ECM) components: Often provided in the form of coating materials for culture vessels, such as Matrigel, vitronectin, or laminin, to support cell attachment and growth in the absence of feeder cells.
   ∘ Other Supplements: Such as non-essential amino acids, antioxidants, and antibiotics/antimycotics to support cell health and prevent contamination.
   The composition of feeder-free maintenance media is part of the common general knowledge in the field of stem cell research. Notable examples include the E8 medium developed by Chen et al. (2011) [Chen, G., Gulbranson, D.R., Hou, Z. et al. Chemically defined conditions for human iPSC derivation and culture. Nat Methods 8, 424-429 (2011). https://doi.org/10.1038/nmeth.1593] and the TeSR medium developed by Ludwig and Thomson (2006) [Ludwig, T.E. & Thomson, J.A. Defined, feeder-independent medium for human embryonic stem cell culture. Nat Methods 3, 637-646 (2006). https://doi.org/10.1038/nmeth902]. These formulations have become widely adopted due to their ability to maintain PSCs in an undifferentiated state under feeder-free conditions.
- The term "neural induction medium" refers to a specialized culture medium designed to induce the differentiation of PSCs into neural progenitor cells, which can further differentiate into various neural cell types, including neurons and glial cells. Neural induction is a critical step in studying neural development, modeling neurological diseases, and exploring potential therapeutic applications.
   Some commonly used neural induction media include supplements such as N2 and B27. N2 supplement provides essential nutrients and factors that support the growth and differentiation of neural progenitors, while B27 is a serum-free supplement that enhances the survival and maturation of neurons. Additionally, other neural induction media formulations may contain Neural Induction Medium (NIM) [Zhang, Xiao-Qing, and Su-Chun Zhang. "Differentiation of Neural Precursors and Dopaminergic Neurons from Human Embryonic Stem Cells." Methods Mol Biol, vol. 584, 2010, pp. 355-366. doi:10.1007/978-1-60761-369-5_19].
- The term "cortical differentiation medium" refers to a specialized culture medium designed to induce the differentiation of pluripotent stem cells (PSCs) into cortical neurons or cortical-like cells. The cerebral cortex, or simply cortex, is the outer layer of the brain responsible for higher cognitive functions, sensory perception, and motor control.
   Cortical differentiation mediums typically contain specific growth factors, signalling molecules, and supplements that mimic the developmental cues necessary for PSCs to differentiate into cortical neurons. These may include factors such as:
   ∘ Neurotrophic factors: Such as brain-derived neurotrophic factor (BDNF), nerve growth factor (NGF), and neurotrophin-3 (NT-3), which promote neuronal survival and maturation.
   ∘ Developmental signaling pathway modulators: Small molecules or inhibitors that regulate pathways involved in cortical development, such as Wnt signalling inhibitors or retinoic acid.
   ∘ Defined serum or serum substitutes: To provide essential nutrients and factors necessary for neuronal differentiation without the variability associated with animal-derived sera.
   ∘ ECM components: Coating materials such as Matrigel, laminin, or poly-L-ornithine that support cell attachment and growth.
   ∘ Other supplements: Including antioxidants, amino acids, and antibiotics/antimycotics to maintain cell health and prevent contamination.
- The term "extracellular matrix (ECM)" refers to a complex network of proteins, glycoproteins, and other molecules that provide structural and biochemical support to surrounding cells. Found in all tissues and organs, the ECM plays a crucial role in cell adhesion, differentiation, migration, and communication. It is composed of various components, including collagen, elastin, fibronectin, laminin, and proteoglycans, which together create a scaffold that helps maintain the integrity and function of tissues. The ECM also regulates a variety of cellular processes by interacting with cell surface receptors and influencing the cellular microenvironment.
- The term "decellularized brain cortical extracellular matrix (ECM)" refers to the ECM obtained from the cortical region of the brain after the removal of all cellular components. The decellularization process involves treating brain tissue with chemical, enzymatic, or physical methods to eliminate cells while preserving the ECM's structure and composition. The resulting decellularized ECM retains the native biochemical cues and structural integrity of the original tissue, making it an excellent scaffold for tissue engineering and regenerative medicine applications. In the context of brain research, decellularized cortical ECM can be used to study cell-ECM interactions, support the growth and differentiation of neural cells, and develop *in vitro* models for investigating brain diseases and testing potential treatments.
- The term "B27 supplement" refers to a serum-free supplement used in cell culture media, particularly in the field of neuroscience and neural cell culture. It was developed to support the growth and differentiation of neural cells, including neurons and glial cells, derived from pluripotent stem cells or other cell sources. The B27 supplement contains a combination of components that provide essential nutrients, antioxidants, and growth factors necessary for maintaining cell viability, promoting cell survival, and enhancing neuronal function *in vitro.*
- The term "N2 supplement" refers to serum-free supplement used in cell culture media, specifically in the culture of neural cells and neural progenitors. It was developed to support the growth and maintenance of neural cells *in vitro,* including neurons and glial cells.
- The term "FGF-2" refers to Fibroblast Growth Factor 2, which is a member of the fibroblast growth factor family of signalling proteins. FGF-2 plays crucial roles in cell proliferation, differentiation, and survival. It is frequently used as a growth factor in cell culture media to maintain the pluripotency of ESCs and iPSCs. It is also used in neural differentiation protocols to induce the differentiation of these stem cells into neural progenitor cells and neurons.
- The term "ROCK inhibitor" refers to a class of small molecule inhibitors that target Rho-associated protein kinase (ROCK). Some examples of ROCK inhibitors include: Y-27632, Fasudil (HA-1077), Ripasudil (K-115), H-1152, GSK269962A, SR3677, Thiazovivin, RKI-1447, Chroman 1 and Blebbistatin.
- The term "inhibitors of the SMAD pathway" refer to small molecule compounds that specifically target and inhibit the intracellular signalling cascade mediated by SMAD proteins. They act by blocking different components of the signalling cascade, including receptor activation, SMAD phosphorylation, or SMAD complex formation. These inhibitors prevent the downstream transcriptional responses that are normally induced by TGF-β family ligands. There are several classes of inhibitors targeting different steps in the SMAD signalling pathway, including TGF-β receptor kinase inhibitors, SMAD protein inhibitors, and downstream signaling inhibitors. Some inhibitors of the SMAD pathway include: SB431542, LY2157299 (Galunisertib), A-83-01, SD-208, RepSox (E-616452), LY2109761, SB525334, LY364947, Pirfenidone, SB505124, GW788388, Vactosertib (TEW-7197), ITD-1, Halofuginone, Sulfasalazine, LY 3200882, TP0427736 HCl, TGFβRI-IN-3, PD 169316, 3,3-Dimethyl-1-butanol, Lycopus Extract, AUDA, BIBF-0775, Ginsenoside Rh4, R-268712 and/or TA-02, Dorsomorphin, Dorsomorphin 2HCl, LDN-193189, LDN-193189 HCl, DMH1, K02288, LDN-212854, MI,347, LDN-214117, SIS3 and/or SIS3 HCl.
- The term "brain disorder, condition, or disease" refers to any abnormality or dysfunction affecting the brain, ranging from neurological disorders to psychiatric conditions. These conditions can involve structural, chemical, or electrical abnormalities in the brain that impact cognitive function, behaviour, emotions, or physical abilities.
- The term "brain disorder, condition, or disease that affects the cortical region of the brain" refers to disorders or conditions that primarily involve or impact the cerebral cortex, the outer layer of the brain responsible for higher cognitive functions such as memory, language, perception, and voluntary movement. Disorders affecting the cortical region may include neurodegenerative diseases (e.g., Alzheimer's disease, Parkinson's disease), neurodevelopmental disorders (e.g., autism spectrum disorders), stroke, traumatic brain injury, and various types of epilepsy.
- The term "candidate compounds suitable for use in a method of treating a brain disorder, condition, or disease" refers to substances that have shown promise in preclinical or clinical studies as potential therapies for addressing abnormalities or dysfunctions in the brain. Examples of candidate compounds may include small molecules, biologics (e.g., antibodies, peptides), gene therapies, or stem cell-based therapies, among others.

### Description of the figures

**Figure 1****:** Characterization of hiPSC line PRPF Control. **A.** Phase contrast image and pluripotency markers. The hiPSC line PRPF Control shows iPSCs-like morphology and stained positive for the pluripotency markers OCT4, SOX2 and SSEA4. **B.** hiPSCs line PRPF Control shows a normal karyotype. Scale bars, 100µm.
**Figure 2****:** Brain organoid protocols. **A.** Protocols' schemes. **B.** Representative images of brain organoids during their development. Non-cortical structures such as CSF fluid cysts are evident in Protocol B organoids. Scale bar, 100µm.
**Figure 3****:** Comparison of brain organoid protocols. **A.** Immunofluorescence analysis. Brain structures are shown by detection of different markers: SOX2, Beta-III-tubulin (TUJ1), MAP2, SATB2, CTIP2, Calretinin (Calret), Synapsin (Syn), Neurofilament (NF). **B.** Transcriptomic analysis of up-regulated cell markers. ± 2-fold change value was applied in the analysis. **C.** Expression arrays. **D.** PCR analysis.
**Figure 4****:** Brain decellularization process scheme.
**Figure 5****:** H&E staining and DNA quantification and identification in electrophoresis gel. Non-remarkably presence of DNA content nor cells is shown in decellularized samples.
**Figure 6****:** Preservation of essential compounds of the cECM. **A.** Total protein and GAGs quantification. **B.** Masson's Trichrome, laminin and tenascin histological staining. Scale bars, 10µm.
**Figure 7****:** Proteome profiling analysis of the decellularized cECM. **A.** List of proteins related to ECM obtained from the analysis. **B.** PANTHER Protein Class. The second most abundant protein class is extracellular matrix protein. **C.** Reactome analysis.
**Figure 8****:** Comparison of Matrigel vs porcine-derived cECM embedded CBOs. **A.** Phase contrast imaging. CBOs embedded in cECM show neural projection. **B.** Immunofluorescence analysis. Brain structures are shown by detection of different markers: SOX2, Beta-III-tubulin (TUJ1), MAP2, SATB2 and CTIP2.
**Figure 9****:** Transcriptomic analysis of Matrigel vs porcine-derived cECM embedded CBOs. **A.** Enrichment analysis in GO-Biological processes terms. **B.** Expression arrays. ± 2-fold change value was applied in these analyses.
**Figure 10****:** CBOs generation process with hiPSC from ALS patients.
**Figure 11****:** CBOs generated from ALS hiPSC linesand controls. **A.** Characterization of hiPSC lines CS29i ALS C9 and CS29i ALS ISOD7. Both lines show positive staining for pluripotency markers such as OCT4, SOX2 and SSEA4, and a normal karyotype. **B.** Phase contrast images of CBOs from PRPF Control line, CS29i ALS C9 line and CS29i ALS C9 ISOD7 line. No significant differences were found among the structures of the three groups of organoids.

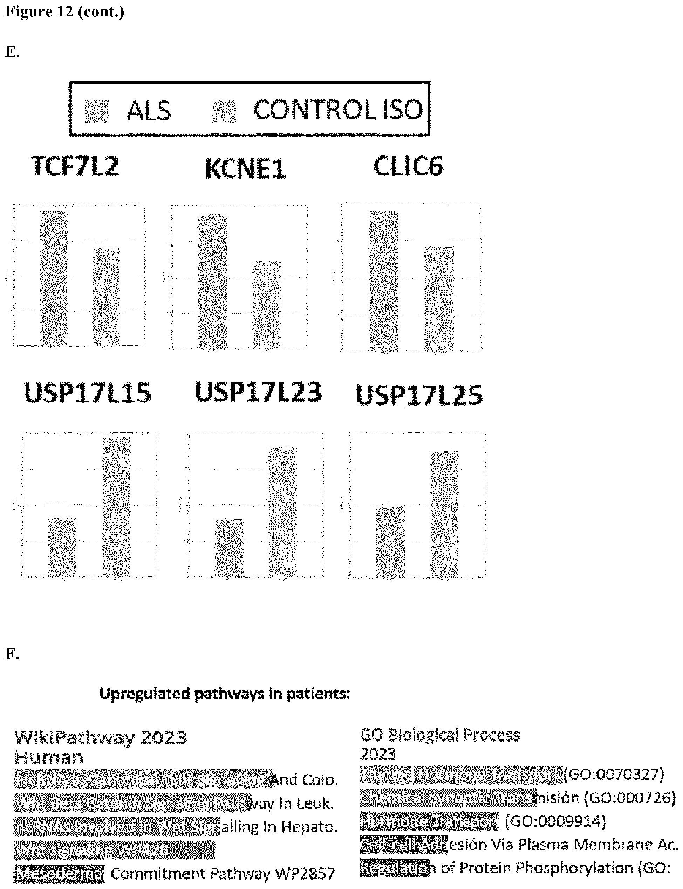

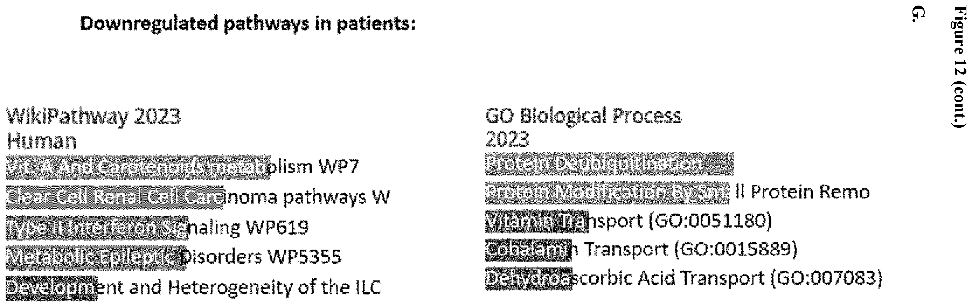

**Figure 12****:** Characterization of cortical organoids from ALS patients. **A** . Detection of neural stem cells (SOX2), astrocytes (GFAP) and neurons (TUJ1) by immunofluorescence. Scale bar: 100 µm. **B.** Immunofluorescence to detect the expression and distribution of neuron markers. Neurofilament (NF), in Green, and doublecortin (DCX) in red. Organoids from ALS express less intracelular NF. Scale bar: 100 µm. **C.** Differentially expressed genes (DEGs) in organoids from C9ORF ALS patients when compared with their isogenic controls (FDR+/-2). **D.** Dot plot comparing organoids from C9ORF ALS patients and organoids from their isogenic controls. Gene names of most differentially upregulated (red) and downregulated (green) genes are marked. **E.** Gene expression of selected genes. TCF7L2, a Wnt signalling effector; KCNE1, a potasium cannel and CLIC6, a chloride Channel are upregulated in ALS organoids. Several ubiquitin peptidases are downregulated in ALS organoids. **F.** Pathways and biological proccess upregulated in organoids from ALS patients. **G.** Pathways and biological proccess downregulated in organoids from ALS patients.
**Figure 13****:** DNA removal and total protein and GAGs quantification of human brain samples. **A.** DNA quantification and identification in electrophoresis gel. Non-remarkably presence of DNA content is shown in decellularized samples. **B.** Quantification of proteins and GAGs shows the preservation of essential compounds of the cECM after decellularization.
**Figure 14****:** Proteome profiling of cECM obtained from ALS patients and healthy donors. Protein classes were analysed with PANTHER online tool (pantherdb.org).

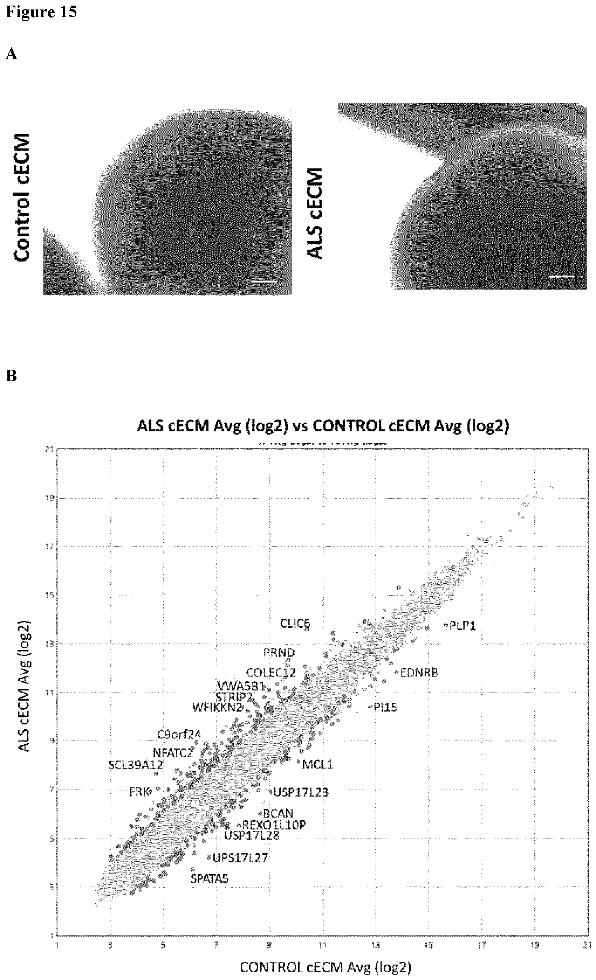

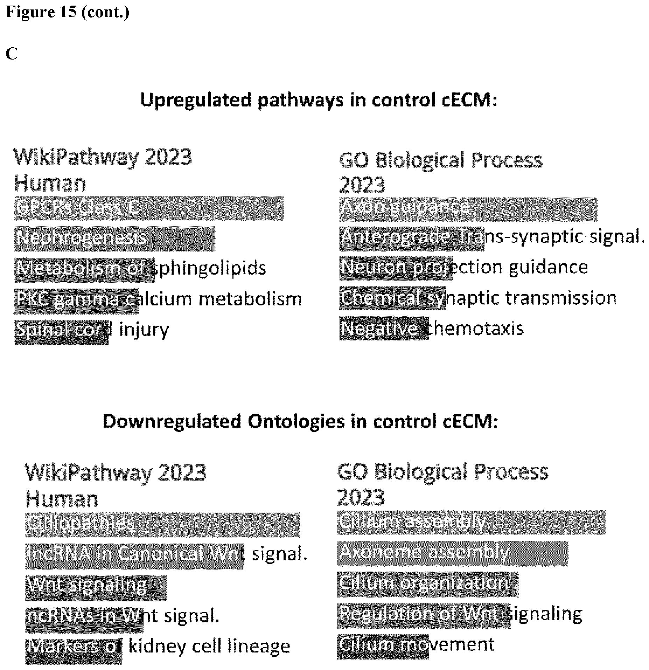

**Figure 15****:** Generation of CBOs with cECM of ALS patients and healthy donors. **A.** Phase contrast images of organoids from hiPSC PRPF control line (derived from a healthy donor) generated by embedding in cECM from motor cortex of ALS patients and with cECM from motor cortex of healthy donors Scale bar: 200 µm. **B.** Dot plot comparing gene expression in organoids generated with cECM of ALS patients and those generated with cECM of healthy donors (FDR+/-2). Gene names of most differentially upregulated (red) and downregulated (green) genes are marked. Several ubiquitin peptidases (USP) are downregulated in ALS cECM embedded organoids. **C.** Pathways and GO terms up- and downregulated in organoids embedded in cECM from ALS patients

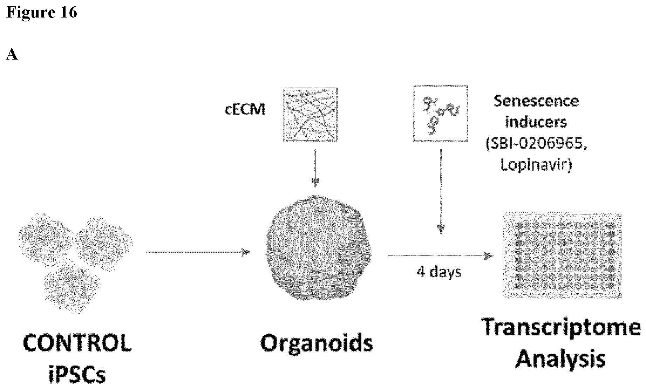

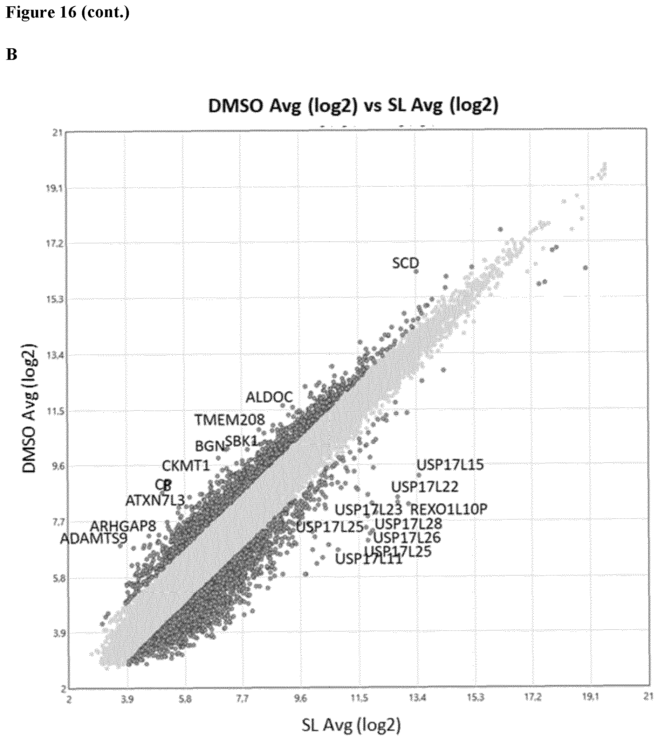

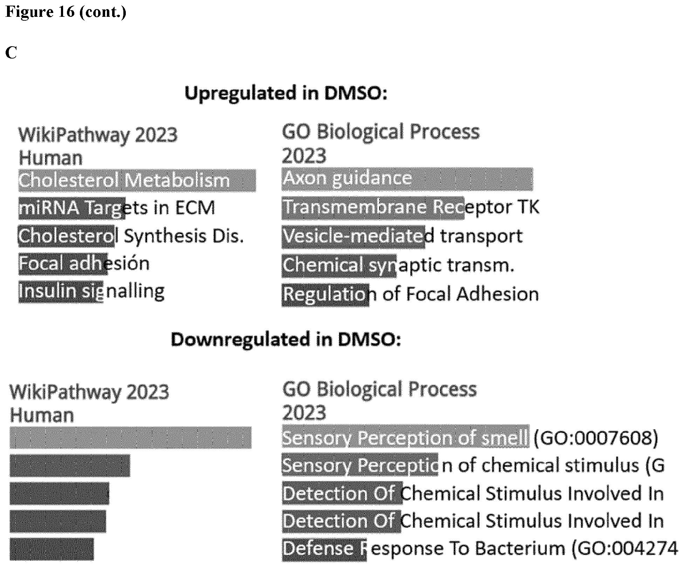

**Figure 16****:** Chemically induced aging of CBOs from healthy individuals. **A.** Aging of organoids from control iPSC was chemically induced by treatment with SBI-0206965 and Lopinavir (SL). **B.** Dot plot comparing gene expression in organoids from control donors treated with DMSO (vehicle control) and organoids where the maturation has been chemically induced by SL treatment. Gene names of most differentially upregulated (red) and downregulated (green) genes are marked. Several ubiquitin peptidases (USP) are deregulated in SL-treated organoids. **C.** Functional enrichment analysis showing up- and downregulated pathways and GO terms comparing aged and not aged control organoids.

### Detailed description of the invention

The present invention is illustrated by means of the Examples set below without the intention of limiting its scope of protection.

### Example 1. Material and Methods

### Culture of human iPSCs (hiPSCs)

A total of three lines of hiPSCs were used for generating organoids. In first place, the line cPRPF31-MiPS4F7 (here named PRPF control) was used as a positive control for the experiments. This cell line is available from the Spanish National Repository and further information about it can be found in https://eng.isciii.es. In second place, two other lines were used, the hiPSC line CS29iALS-C9n1 with a C9orf72 mutation and the genetically corrected hiPSC line, CS29iALS-C9n1 ISOD7. The first one is derived from fibroblasts of an amyotrophic lateral sclerosis (ALS) patient and the second is derived from the first hiPSCs line. These cell lines are available from Answer ALS-Cedars Sinai and further information can be found in https://csbiomfg.com. All the lines were maintained on Matrigel-coated plates and cultured in mTeSR^{™} Plus medium (Stemcell Technologies, Cat. #100-0276) supplemented with mTeSR plus supplement 5X (Stemcell Technologies, Cat. #100-0275). The culture followed standard procedures in 5% CO2 incubator at 37°C and the medium was changed every second day. The use of hiPSCs was approved by the Andalusian Ethical Committee of Research with Biological Samples of and Embrionyc Origin and Similar Cells (PR-02-2016).

### Characterization of pluripotency in the hiPSCs lines

Pluripotency in the three lines of hiPSCs was confirmed before organoid generation. For this purpose, the expression of the nuclear transcription factors OCT4 and SOX2 in the nucleus, and the surface marker SSEA4, was assessed. First, iPSCs were fixed in 4% paraformaldehyde (PFA, PanReac, Cat. #252931.1211) for 15 minutes at room temperature (RT). After washing twice with phosphate buffered saline (PBS, Sigma-Aldrich, Cat. #D8537), the cells were permeabilized in PBS 0.1% Triton X-100 (Sigma- Aldrich, Cat. #T8787) and blocked in PBS with 1% bovine serum albumin (BSA, Sigma-Aldrich #A8806) for 30 minutes at 37°C. Then, they were incubated with the primary antibodies, previously diluted in PBS with 0.1% BSA, for 30 minutes at 37°C. Subsequently, the cells were incubated with the secondary antibodies for 30 minutes at 37°C and they were maintained in PBS at 4°C until their use. The primary and secondary antibodies used in this experiment and their working dilution are listed in Annex II. Furthermore, the cell cultures were observed by contrast phase microscopy and normal karyotype was also probed before using the cells for organoids generation. The karyotype with Bands-G of the cell cultures was performed by the Andalusian Public Health System Biobank. For this, the cells were first treated with colcemide and later fixed with methanol/acetic.

### Preparation of the brain cECM by decellularization

The brain tissues were obtained from two different sources. First, for setting up the decellularization protocol in this project, the motor cortex of a porcine brain was used. All the analyses related to the cECM were first set up in porcine material due to difficult availability of human material. Next, human brain tissues were obtained from both brain samples of ALS patients and healthy subjects. The technical report of the human brain tissues is shown in **Table 1.** In these cases, the samples were obtained from the motor cortex and the visual cortex of the patients' brains. Briefly, frozen brain tissues were thawed at RT and agitated in buffer solution with different solvents such as distilled water, trypsin/ ethylenediaminetetraacetic acid (trypsin/EDTA, Lonza, Cat. #BE17-161E), Triton X-100 with ammonium hydroxide (Sigma-Aldrich, Cat. # 1336-21-6), 1 M sucrose solution (Sigma-Aldrich, Cat. #S0389), sodium dodecyl sulphate (SDS, Fisher Scientific, Cat. #BP166-100), ethanol (Sigma- Aldrich, Cat. #E7148), PBS and penicillin-streptomycin (P/S, Sigma-Aldrich, Cat. #P0781). Then, whole decellularized brain tissues were lyophilized and stored at 4°C. For its use, the cECM was prepared by solubilizing the decellularized samples with 4 mg/ml pepsin in 0.02M HCl and stirred at RT for two days. In the organoid culture, the concentration of cECM was 40mg/ml and it was mix with Matrigel (Corning, New York, NY, USA, Cat. 354277) for a final concentration of 400 µg/ml.

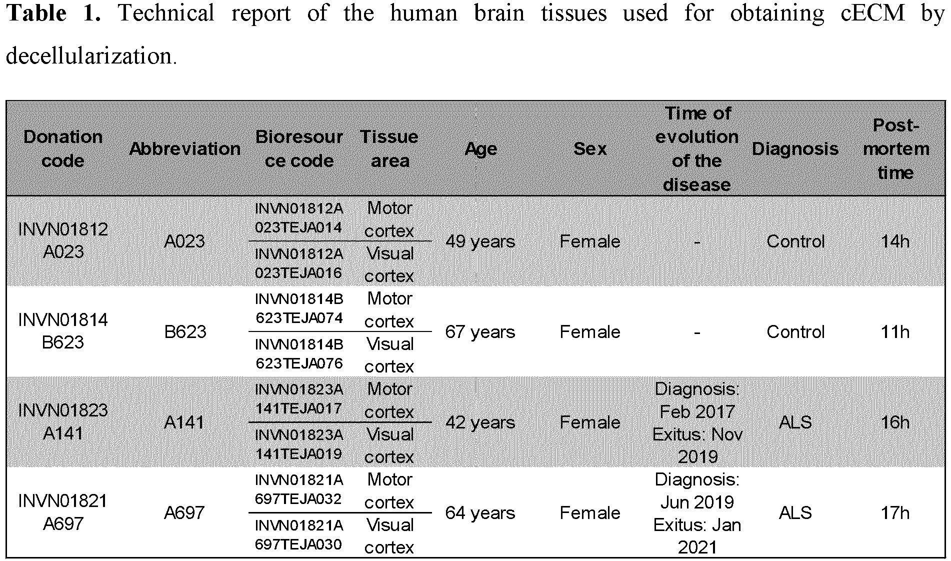

### Characterization of the brain cECM

For the characterization of decellularized porcine brain cECM, two histological analyses were performed in order to confirm the removal of cells and the permanence of cECM components in this sample. On the one hand, H&E staining of sectioned samples was performed to assess the absence of cells in the decellularized cECM. On the other hand, Masson's Trichrome (MT) was performed for confirming the preservation of collagen after the decellularization process. In addition, immunostaining of laminin and tenascin was also performed to check the preservation of these proteins in the decellularized samples. For the analysis, the samples were first fixed in 4% PFA and then sent to Histology and Microscopy Core Facilities of the Andalusian Center of Molecular Biology and Regenerative Medicine (CABIMER) for being sectioned to 6µm thickness and further processed for immunohistochemistry.

The removal of cellular components in all the decellularized samples was confirmed by DNA quantification and DNA electrophoresis in agarose. In both cases, the total DNA was obtained using a DNA extraction kit (DNeasy^{®} Blood & Tissue Kit, Qiagen, Cat. #69504) following the manufacturer's instructions. First, for quantifying DNA content, absorbance at 260nm was measured using QubitTM (Qubit 3 Fluorometer, Thermo Fisher Scientific, Cat. #Q33216). For electrophoresis, an 1% agarose gel was prepared using TAE 1X. The gel was run for 1h and 20 minutes at 60V.

For confirming the presence of general proteins in the decellularized cECM, a protein extraction and quantification was performed. The samples were placed in ice during all the procedure. A mixed solution of 50µl of RIPA buffer (Sigma-Aldrich, Cat. #R0278) and 0.5µl of 100X protease inhibitor (Sigma-Aldrich, Cat. #P8340) per sample was used for breaking the cellular membrane and keeping the stability of the proteins. After vortex, the samples were incubated in ice for 10-15 mixing every 5 minutes. Then, the samples were centrifugated for 15-20 minutes, 13000rpm and at 4°C. The supernatant was transferred to a clean 1,5 ml microtube and the quantification was performed using Qubit^{™} and following the manufacturer's instruction. In addition, GAGs content was measured in both non-decellularized and decellularized brain cECM using a kit for colorimetric assay (Blyscan^{™}, Biocolor Ltd, Cat. #B1000) according to manufacturer's instructions. The results of the analysis were obtained using a the multiwell plate reader Spark10M^{®} (TECAN, Cat. #R_0039).

A proteome profiling analysis was also performed to identify main proteins present in the decellularized samples. The proteins extracted from decellularized cECM were sent to the Proteomics Unit of the Institute of Biomedicine of Seville (IBiS) for performing a liquid chromatography-mass spectrometry (LC-MS) technique. For processing the obtained data, the software Proteome Discoverer was used with a value of FDR>1%.. The data treatment has been produced using the online tools Panther (pantherdb.org) and Reactome (reactome.org). For these analyses, the raw data was modified to remove the proteins added for the LC-MS analysis (pepsin and trypsin) and those proteins identified with a score value equal than 0.

### Generation of cortical brain organoids (CBOs)

First, CBOs from the PRPF Control hiPSCs were generated following two different protocols. The first protocol described in this section is referred as protocol A. The second is referred in this project as protocol B. However, both follow a similar stepwise procedure with some differences among them.

In both cases, hiPSCs colonies were isolated and disaggregated to obtain single cells with Gentle Cell Disociation Reagent (GCDR, Stemcell Technologies) for 8 min at 37°C. Next, the dissociating agent was inactivated adding mTeSR plus Medium and next transferred in a 15-ml conical tube for centrifuging at 300g for 5 min. The supernatant was discarded, and the pellet was resuspended in mTeSR plus Medium. Then, cell counting was performed and, depending on the protocol, the needed volume of cell suspension was mixed with mTeSR plus Medium supplemented with 10 µM Rho-associated protein kinase inhibitor (ROCK-i) Y-27632 (Tocris Bioscience, Cat. #1254).

In protocol A, for the formation of EBs, 9000 cells/well were seeded in mTeSR plus Medium with 10 µM ROCK-I in 96 well U-bottom low attachment plate (Corning, Cat. #CLS7007). On day 2, the inhibitors were added to the culture conditions as follows. Dorsomorphin (Sigma-Aldrich, Cat. #P5499) and SB431542 (Tocris Bioscience, Cat. #1614) were added to reach a final concentration of 5 µM and 10 µM, respectively. On day 6, for the induction to neural fate, the EBs were transferred to 24 low attachment wells (Corning, Cat. #3473) with neural induction media (NIM) containing Dulbecco's modified Eagle Medium (DMEM):F12 (Gibco by Thermo Fisher Scientific, Cat. #21331020), Glutamax (Gibco by Thermo Fisher Scientific, Cat. #35050-061), 1X N2 supplement (Gibco by Thermo Fisher, Cat. #17502-048), minimum essential media non-essential amino acids (MEM-NEAA) (Sigma-Aldrich, Cat. #P4999) and 1 µg/mL heparin 1000 UI/mL (Rovi, Cat. #641747). Additionally, the NIM contained the same final concentration of the inhibitors Dorsomorphin and SB431542previously indicated. The embryonic bodies (EBs) were fed every other day with this medium for 4-5 days. On day 10, the formed organoids transferred to a 6-well low attachment plates are embedded in Matrigel and expanded in differentiation media with a 1:1 mixture of DMEM/F12 and Neurobasal (Gibco by Thermo Fisher Scientific, Cat. #21103-049) supplemented with 2X B27 supplement without vitamin A (Gibco by Thermo Fisher Scientific, Cat. #12587-10), 1X N2 supplement, 1X Glutamax, 1X MEM-NEAA, 0.09% 2-mercaptoethanol (Gibco by Thermo Fisher Scientific, Cat. #21985-023), 1X P/S (Sigma-Aldrich, Cat. #P4333), and 0.025% insulin (Sigma-Aldrich, Cat. #I9278). The inhibitory molecules were withdrawn from this point on. For 4 days, the organoids were incubated at 37°C. Next, on day 15, they were grown with the same differentiation media but adding B27 supplement with vitamin A (Gibco by Thermo Fisher Scientific, Cat. #17504-044) for maturation. This medium was changed every three days and the organoid culture was maintained at 37°C in agitation.

The protocol B shares the majority of steps with the protocol A, thus only the modifications are next mentioned. In this protocol, before forming EBs, PLGC microfilaments were produced. 18000 cells/well were seeded in ultra-low binding 96-well plate for the generation of EBs in a 1X microfibers solution in mTeSR plus Medium with ROCK-i. During this protocol, no inhibitors were added to the culture. However, the molecule CHIR (Axon Medchem, Cat. # 252917) was added in a final concentration of 3µM two days after embedding the organoids in Matrigel and was maintained in the culture medium for 2 days. Except for these modifications, the rest of the process was performed the same as previously mentioned in protocol A, excluding the dual-SMAD inhibitory molecules SB431542 and Dorsomorphin.

### RNA isolation

At least three organoids of each condition were collected and pooled for total RNA isolation using an RNA extraction kit (RNAeasy Mini Kit; #74004, Qiagen) according to manufacturer. Qubit^{®} 4.0 was used for measuring the absorbance of the isolated RNA samples at 260nm for calculating RNA concentration.

### RT-PCR analysis of gene expression in organoids

Reverse-strand cDNA synthesis was performed following a reverse transcription kit (SuperScript^{™} II Reverse Transcriptase, Thermo Fisher Scientific, Cat. #18064014). These cDNA samples were used for RT-PCR analysis using MyTaqTM DNA polymerase (Bioline, Meridian Life Science, Cat. # BIO-21105). Several RT-PCRs were performed using primers for a variety of pluripotent and germ layer identities and especific cell types such as mature neurons and astrocytes. PCR conditions and number of cycles changed depending on the primer pair used. In general, the cycles (30-38) were run at 94°C for denaturation for 30s, 55-60°C annealing for 45s depending on the primer pair, and 72°C extension for 30s. The primer pair used with their specific conditions are listed in **Table 2.** RT-PCR products were analysed by electrophoresis in 1.5% agarose TAE 1X gel using 10µl of Syber Green for DNA staining (Invitrogen, Cat. #S7563,).

**Table 2. Primers.**

| **Primer** | **Forward primer** | **Reverse primer** |
|---|---|---|
| AFP | ATTTAAACTCCCAAAGCAGCAC | TAAACCCTGGTGTTGGCCAG |
| Brachiury | TTTCAAAGCAGTGGAGGAGCACAC | ACTGCATCATCTCCACAGTTGGGT |
| CTIP2 | CTCCCTTTGGATGCCAGTGTCA | GGCTCCAGGTAGATGCGGAAG |
| DCX | CATCCCCAACACCTCAGAAG | GGAGGTTCCGTTTGCTGA |
| EMX2 | GGGATCCGTCCACCTTCTAC | CTCAAAGGCGTGTTCCAGCC |
| FOXG1 | AGAAGAACGGCAAGTACGAGA | TGTTGAGGGACAGATTGTGGC |
| GAPDH | TGCACCACCAACTGCTTAGC | GGCATGGACTGTGGTCATGAG |
| GATA4 | CTCCTTCAGGCAGTGAGAGCC | GGTCCGTGCAGGAATTTGAGG |
| Laminin | CTTCTGGAGCAGATGAGGCAGCACATG | TGCCTCCTGCAGCCGAGCACGAAG |
| MAP2 | TAACCAACCACTGCCAGACCTGAA | GCCACATTTGGATGTCACATGGCT |
| PAX6 | CCGGCAGAAGATTGTAGAGC | CGTTGGACACGTTTTGATTG |
| SATB2 | CAAGAGTGGCATTCAACCGCAC | ATCTCGCTCCACTTCTGGCAGA |
| SOX2 | AGAACCCCAAGATGCACAAC | ATGTAGGTCTGCGAGCTGGT |
| Synapsin 1 | GCCAATGGTGGATTCTCTGT | GTCCTGGAAGTCATGCTGGT |
| TH | TCATCACCTGGTCACCAAGTT | GGTCGCCGTGCCTGTACT |
| Tuj1 | AGTCGCCCACGTAGTTGC | CGCCCAGTATGAGGGAGAT |
| v-GLUT | CACGTGGTGGTGCAGAAA | CGTGTATGAGGCCGACAGT |
| ACTA2 | AAATACTCTGTCTGGATCGGTGGCT | CACATAGGTAACGAGTCAGAGCTTTG |

### Transcriptomic analysis for organoids

Total RNA of pooled organoids was analysed in the Genomics Unit of the Andalusian Center of Molecular Biology and Regenerative Medicine (CABIMER) for transcriptomic analysis. First, the RNA quality was checked using the Bioanalyzer 2100 (Agilent, Cat. #G2939BA) and all samples had an RNA Integrity Number (RIN) higher than 9. For the analysis, cDNA was synthesized, biotin-labelled and hybridized with independent Human Clariom-S Microarrays, (Affymetrix, Cat. #902927) following Affymetrix protocol. Affymetrix GeneChip Scanner 3000 7G was used for scanning the microarrays and the outcome was analyzed with the Affymetrix GeneChip Command Console 2.0 software.

The microarray data were further analysed using three analysis databases. Firstly, Affymetrix Transcriptome Analysis Console (TAC, Affymetrix) was used for the study of genes separately thus allowing the focus on the differential expressed gene markers of interest. This analysis was performed with a ±2 gene-level fold change. No P-value was considered due to the shortage of samples that they could afford to analyse in terms of availability of organoids. Secondly, EnrichR was used for performing functional enrichment analysis. Lastly, the bioinformatic tool DAVID (http://david.abcc.ncifcrf.gov) was used for functional interpretation of the genes derived from the microarray.

### Immunofluorescence of cortical brain organoids

For immunofluorescence assays, CBOs serial sections were produced in Histology and Microscopy Core Facilities of the Andalusian Center of Molecular Biology and Regenerative Medicine (CABIMER). First, the samples were washed with PBS twice and then they were blocked and permeabilized in 0.1% Triton X-100 and 10% Donkey serum (DKS, Sigma, Cat. #D9663) in PBS. Next, they were washed with PBS and an antigen retrieval step was performed using 10mM citrate buffer (Thermo Fisher Scientific, Cat. #J63950). Section samples were then incubated overnight at 4°C with primary antibodies in PBS containing 10% DKS. After three washings with PBS 1% DKS, the samples were incubated for 1h at RT with the secondary antibodies and Hoechst (Hoechst 33342 Staining Solution, Miltenyi Biotec S.L, Cat. #130-111-569,) in PBS 10% DKS. The samples were then washed three times with PBS and once in distilled water before finally mounting them for microscopy on glass coverslips with ProLongTM Gold Antifade Mountant (Thermo Fisher Scientific, Cat. #P36930). The working dilutions of the primary and secondary antibodies used are listed in **Table 3.** For the obtention of fluorescence images, a Nikon Eclipse Ti-S microscope was used. Image J 1.53t software developed at the National Institutes of Health and the Laboratory for Optical and Computational Instrumentation (LOCI, University of Wisconsin, Madison, WI, USA) was used for processing the obtained images.

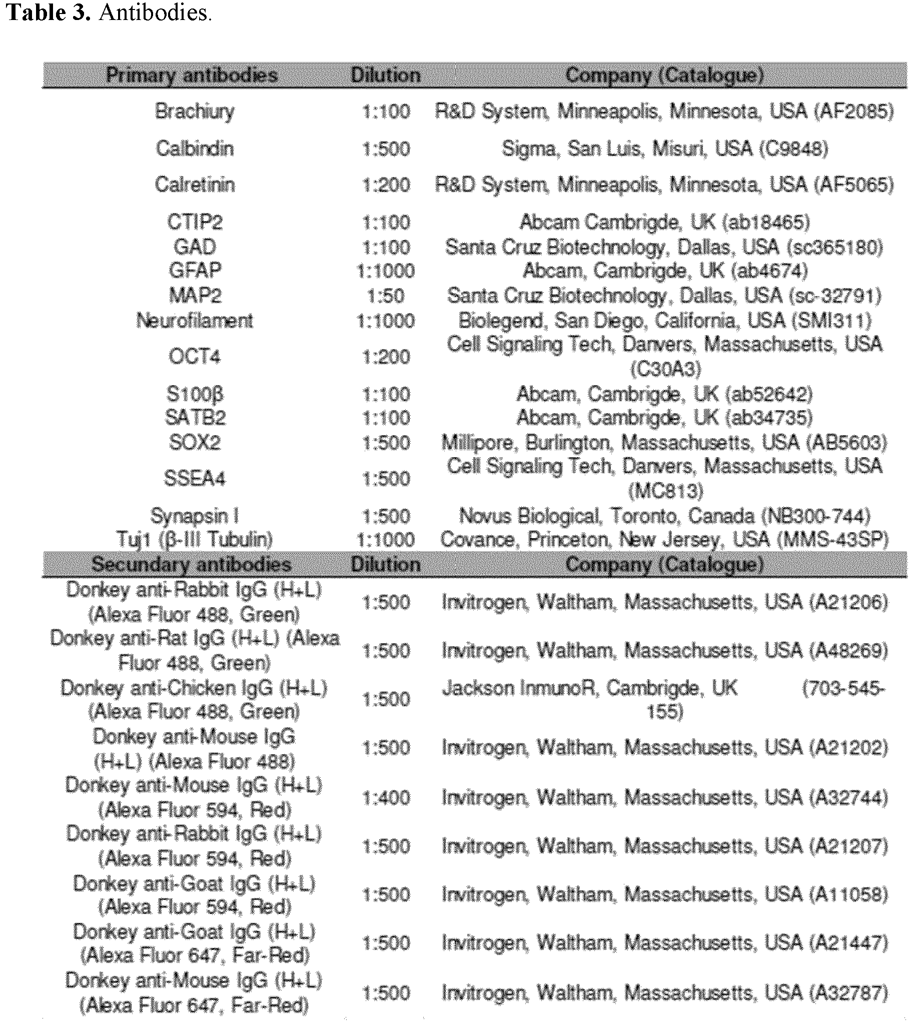

### Example 2. Results

### Example 2.1. A guided protocol with inhibitory molecules gives rise to organoids with more cortical markers and less contamination with non-ectodermal layers

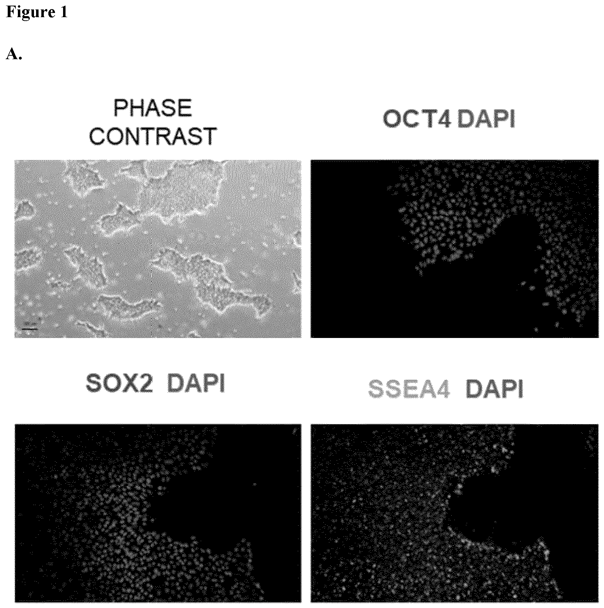

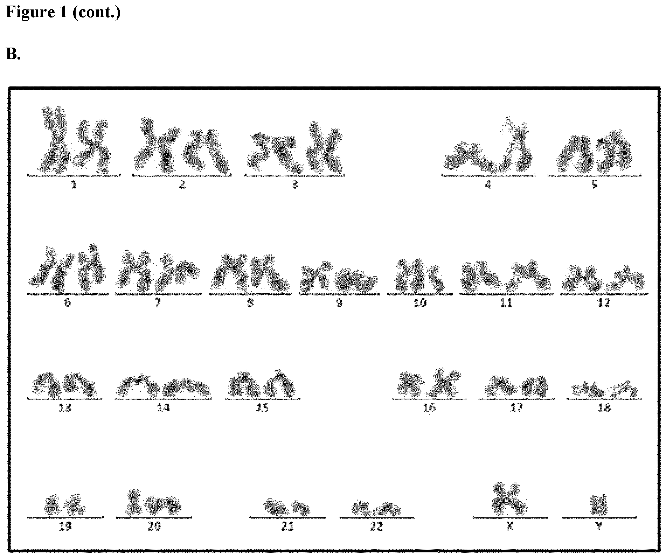

In order to optimize brain organoid (BO) generation, the inventors generated organoids by using two different protocols. For this purpose, they used the hiPSC line cPRPF31-MiPS4F7 from a healthy donor (PRPF Control). First of all, before using this hiPSCs line for generating BOs, pluripotency and normal karyotype were analysed. For characterizing the pluripotency of the hiPSC, the cell cultures were observed by contrast phase microscopy and an immunofluorescence assay was performed. The hiPSC line showed iPSCs-like compact colonies with defined and regular borders **(****Figure 1A****)** and stained positive for pluripotency markers such as OCT4, SOX2 and SSEA4, confirming their pluripotency. The karyotype was confirmed normal **(****Figure 1B****).** Thus, the inventors proceeded to the generation of BOs from this cell line.

They followed two protocols, named in this project as protocol A and protocol B, classified as guided and unguided protocols respectively. The most important differences among them is the addition of dual-SMAD inhibitory molecules of BMP and TFG-β pathways (SB431542 and Dorsomorphin) in protocol A and the addition of fibrous microscaffolds in protocol B **(****Figure 2A****).** 48 hours from the starting point of the protocols, the inventors observed that the suspended cells were grouped in spherical embryonic bodies (EBs). Then, 6 days from the start, the EBs showed a lighter and defined outer border, and at this point the neural induction media was added to the organoids. At day 10 from the start and after embedding the organoids in Matrigel, they displayed an expanded neuroepithelium with neurobuds. These BOs were maintained in culture for 2 months approximately. At this point, the presence of several human brain tissue and cell types was characterized by immunofluorescence analysis, transcriptomic analysis, and RT-PCR.

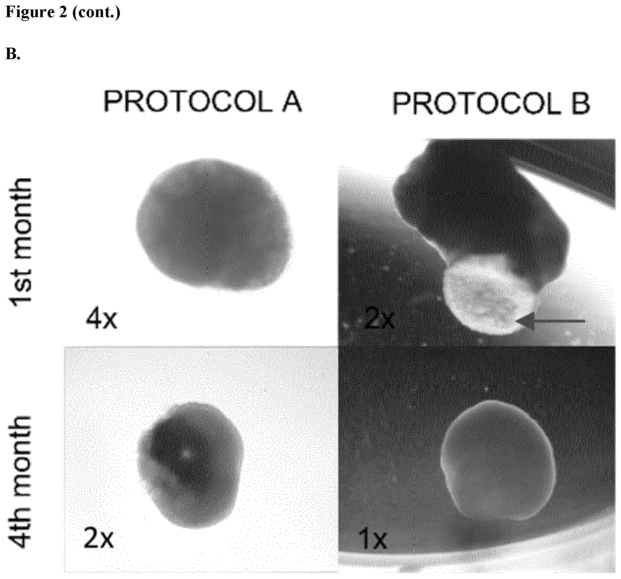

Under phase contrast microscopy and under human eyesight, organoids obtained using protocol B showed brain structures different from the cortex, such as cerebrospinal fluid (CSF) cysts **(****Figure 2B****,** arrow). Nevertheless, not all organoids generated from protocol B showed these structures, causing a heterogeneous group of organoids. At early stages, around 15 days, the organoids formed a continuous neuroepithelia around the organoid structure.

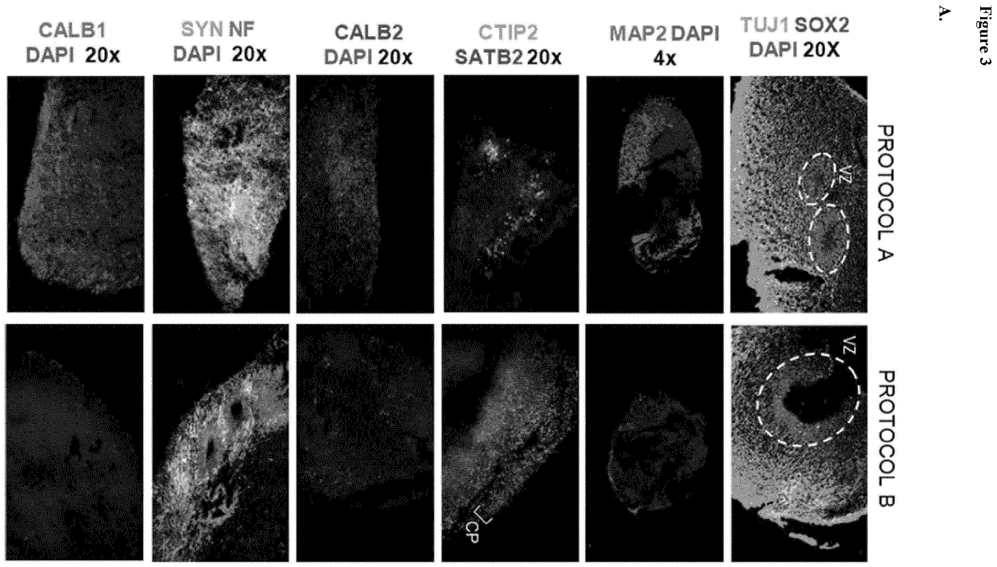

A ventricle-like structures were identified in BOs from both protocols, resembling the ventricular zona (VZ) of a human brain **(****Figure 3A****,** circled around). The VZs were delimited by many neural stem cells (NSCs) stained positive for the stem cell transcription factor SOX2. Furthermore, cerebral cortex, a more mature structure in the human brain, was also displayed in the organoids. The organoids showed a well-organized cortical plate with more mature neuronal cell types located in the outer region of the VZ, staining positive for neuron-specific beta III tubulin (TUJ1) and microtubule-associated protein 2 (MAP2). Early-born SATB2+ and late-born CTIP2+ cortical neurons formed rudimentary cortical layers within the organoids defining the cortical plate (CP). Even though organoids from both protocols stained positive for all the markers before mentioned, there are some differences in the expression of these markers between the protocols.

The ventricles observed in organoids from protocol B were generally bigger than the ones found in protocol A. Furthermore, the BOs obtained following protocol B show a more structured SATB2/CTIP2 cortical layering than those found in protocol A. In contrast, they found a higher expression of MAP2, calbindin (CALB1) and calretinin (CALB2) in protocol A, indicating more maturity within these organoids and identifying cortical γ-aminobutyric acid (GABA) interneurons. In addition, the marker synapsin (SYN), located in the synaptic terminals of neurons, also showed a higher expression in protocol A organoids **(****Figure 3A****).**

A transcriptomic analysis was next performed to study the difference and similarities among organoids obtained following both protocols. A list of differentially expressed genes among the organoids from the two protocols was obtained establishing a ±2-fold change threshold. A functional enrichment analysis was performed **(****Figure 3B****),** showing that the upregulated genes in protocol A in comparison to protocol B were enriched for GO (gene ontology) terms related to neuronal differentiation. Among these results, the inventors noticed that the first identification indicates a greater representation of glutamatergic neurons from the dorsolateral prefrontal cortex. Notwithstanding, the upregulated genes in organoids from protocol B in comparison to protocol A were enriched for GO terms related to non-neuronal fates, mostly related to cardiac tissue. This analysis shows that, effectively, the dual-SMAD inhibitory molecules SB431542 and Dorsomorphin added in protocol A, have a positive effect on the organoids enriching them in neuroectoderm tissue. Those organoids generated without these molecules in protocol B are contaminated with other non-ectodermal layers, developing other tissues like cardiac. On the other hand, a gene-by-gene analysis was performed to seek genes differentially expressed among the different organoids **(****Figure 3C****).** The inventors found that, in protocol A organoids, the most remarkable differential expression was found in the genes FOXG1 and EMX2, both representing the presence of cortical regions within these BOs. The neural progenitor cells (NPCs) cortical marker PAX6 was also more expressed in protocol A organoids. Furthermore, markers for motor cortical layer, such as CTIP1 and FEZF2, were highly expressed in protocol A. In addition, mature neurons markers such as MAP2 and CALB2 showed a higher expression in these organoids, and so did vGLUT, a glutamatergic neuron marker. Conversely, the most differentially expressed gene among organoids from protocol B in comparison to protocol A was alfa-fetoprotein (AFP), which represents endodermal fate.

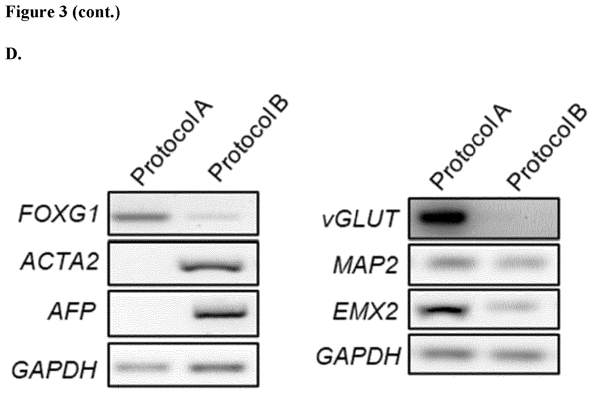

In addition, other non-ectodermal genes were expressed in these organoids, showing a higher representation of mesodermal markers such as actin alpha 2 smooth muscle gene (ACTA2) and actin alpha cardiac muscle 1 gene (ACTC1). Some of these findings were confirmed by RT-PCR analyses **(****Figure 3D****).** Overall, these results suggest that those organoids generated from protocol A display a more cortical identity and those generated following protocol B showed a higher expression of mesoderm and endoderm markers. Even though both kind of BOs contained NPCs, GABAergic neurons, glutamatergic neurons, and glial cells, those obtained following the guided protocol A showed a higher expression for all of these markers. Therefore, the most adequate protocol for the generation of cortical BOs is the guided protocol A and the inventors henceforth name the organoids generated in the following experiments as cortical brain organoids (CBOs).

### Example 2.2. The addition of porcine brain derived extracellular matrix provides more mature cortical brain organoids

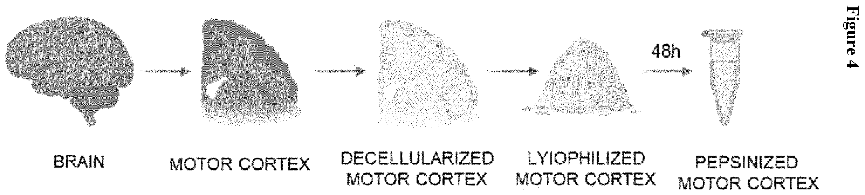

After the comparison between the two protocols, the inventors decided to use protocol A for an improved generation of CBOs in their laboratory. A further step to improve the CBO model was to embed the organoids in porcine motor cortex ECM (cECM) instead of Matrigel in order to assess whether this has a positive effect on the organoid development and maturation. For decellularizing the motor cortex of porcine brain, several detergents were used to remove the cellular component from the tissue, due to the ability of these agents to solubilize the cell membrane **(****Figure 4****).** Brain ECM is primarily composed of glycosaminoglycans (GAGs), hyaluronic acid and proteoglycans. Therefore, the decellularization process should allow the preservation of these components within the ECM whilst removing the DNA and cellular content. To verify the effectiveness of the decellularization process, several studies were conducted.

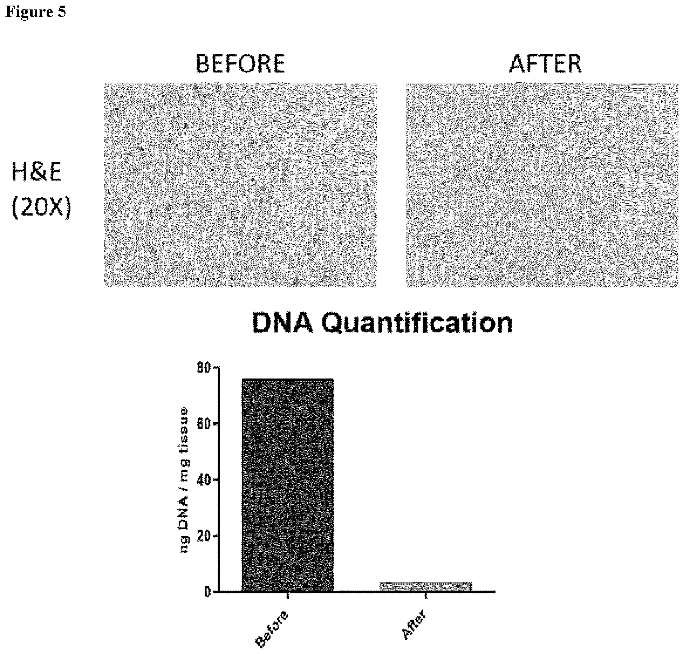

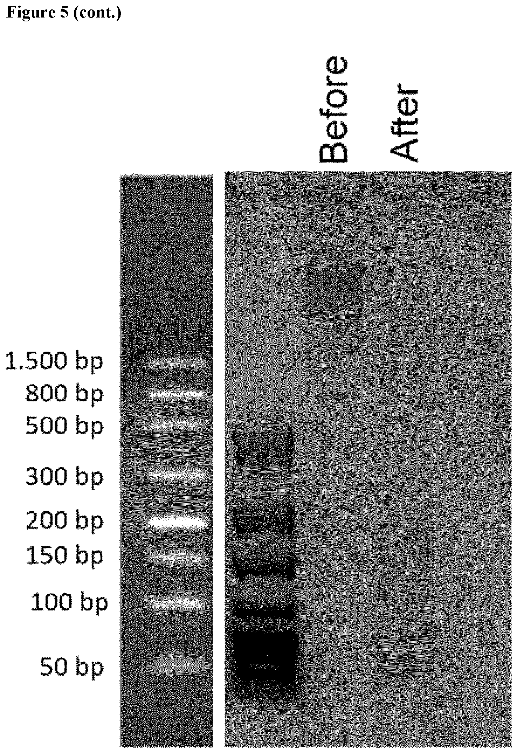

First, the efficiency of the decellularization was assessed by DNA quantification for validating DNA removal and hematoxylin and eosin (H&E) histological staining for validating cellular material removal **(****Figure 5****).** The DNA content was reduced in a 95.27% during the decellularization process. In addition, DNA removal was also confirmed by analyzing both the native and decellularized brain samples in an electrophoresis gel. Histological evaluations with H&E validated the efficacy of decellularization by showing the presence of nuclei in the native brain samples but their absence in the decellularized samples.

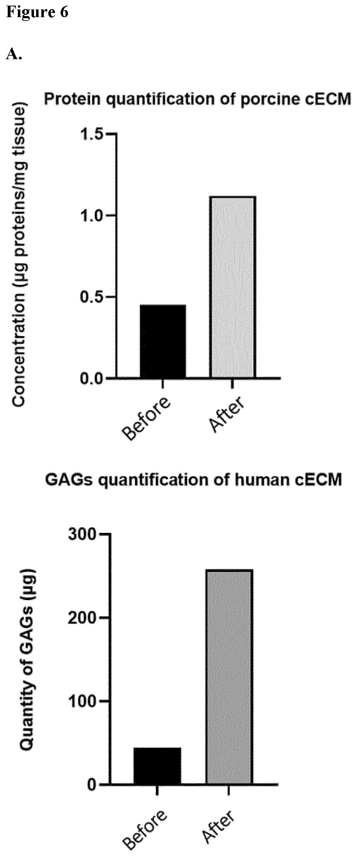

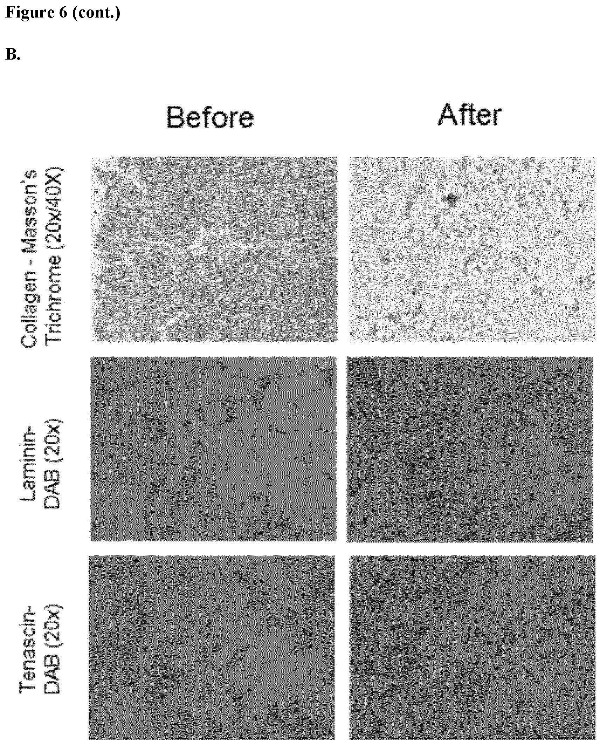

The efficiency of this process was also assessed in terms of ECM components, total protein, and glycosaminoglycans (GAGs) preservation in the decellularized sample **(****Figure 6A****).** For this purpose, collagen, laminin, and tenascin staining were performed, which successfully validated the preservation of these proteins after decellularization **(****Figure 6B****).** In addition, GAGs and protein quantification was performed for evaluating the preservation of these essential ECM components. The results of these analysis show the correct preservation of both components after de decellularization. It is noticeable that in the pepsinized sample both the concentration of GAGs and proteins are higher than in the non-decellularized brain. This could be due to the process of decellularization itself, since it includes a lyophilization process which concentrates the proteins in the sample by eliminating water, and also to pepsinization, which includes the addition of pepsin, an enzyme that can also be part of the proteins that their analysis measured.

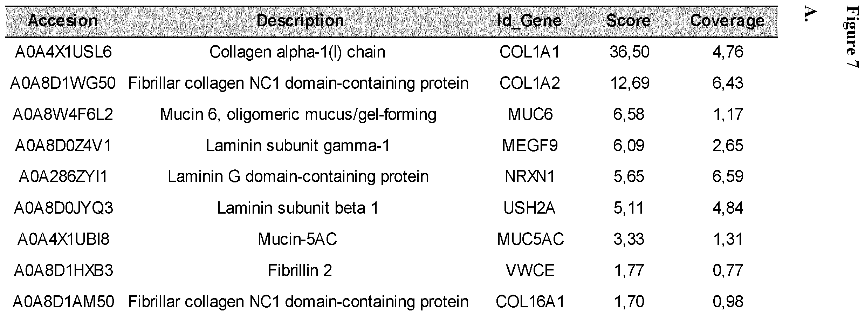

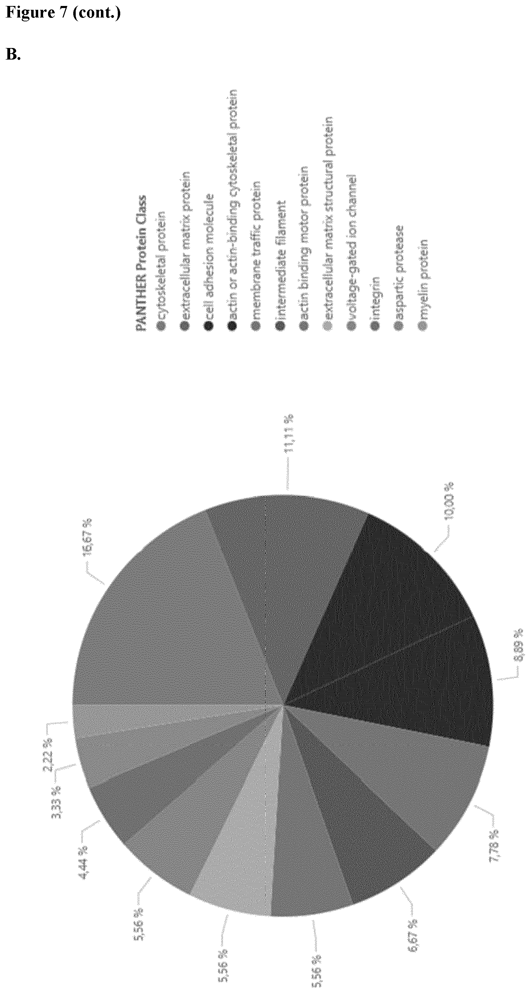

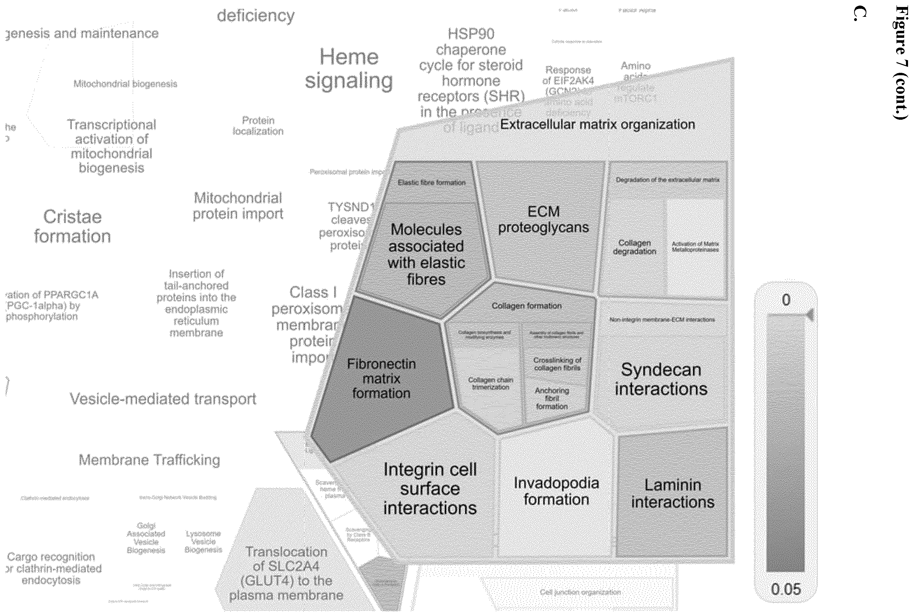

Once the presence of proteins in the cECM was validated, a proteomic profiling analysis was performed to specify which proteins were mostly present in this sample. After treating the raw data and using bioinformatics tools, they obtained a profile of the present proteins in the cECM. They obtained a list which contained several proteins related to ECM **(****Figure 7A****).** It was remarkable that the second most abundant protein class were extracellular matrix proteins, showing that the cECM contains specific ECM proteins. Extracellular matrix structural proteins were listed in a lower position of this same analysis **(****Figure 7B****,** red box). Another class of proteins related to ECM, extracellular matrix structural proteins in this case, appeared again below in this same analysis. In addition, using Reactome database they observed that a significant part of the represented group of proteins with a p-value < 0.05 it is also related to extracellular matrix organization **(****Figure 7C****).**

After characterizing the cECM obtained after decellularization, the inventors generated another two groups of organoids from the hiPSCs line PRPF control by following protocol A. In this case, they compared conditions: organoids embedded in Matrigel and organoids embedded in the porcine cECM. Their development was followed by light microscopy, and they were characterized by immunofluorescence and transcriptomic analysis. Under phase contrast microscopy, no significant differences were noticed between both groups of organoids, which showed the same neural structures during the development and grew at similar pace. Nevertheless, organoids embedded in cECM showed higher neuronal projections to the outer zone of the CBOs **(****Figure 8A****).**

Immunofluorescence arrays showed differential expression of several markers among the organoids **(****Figure 8B****).** VZ structures were evenly found in both groups, being delimited by NSCs that stained positive for SOX2, and more mature neuronal cell types were equally located in the outer region of these VZ staining positive for TUJ1. In organoids embedded in cECM, the expression of the marker MAP2, a marker for mature neurons in the cortical-like plate, was higher. They observed that these organoids showed a better cortical organization than the ones embedded in Matrigel, showed by their expression of CTIP2, an early expressed marker involved in specifying subcortical projection neuron, and SATB2, a later expressed marker which defines corticocortical projecting neurons. These results show a positive effect of the cECM on the organoid generation process, showing that in this way, the organoids display a more developed cortical structure.

To seek which genes were upregulated and downregulated among the CBOs, a transcriptomic analysis was performed. A gene list was obtained establishing a ±2-fold change threshold in the analyses. The functional enrichment analysis **(****Figure 9A****)** showed that in organoids embedded in cECM, the upregulated genes were enriched for synaptic processes, meanwhile there was a downregulation of genes for cell cycle processes and cell division genes, indicating a higher focus on differentiation and minor focus on proliferation. To seek genes involved in neuronal development, synapsis processes and neuronal maturation, they performed a gene-by-gene analysis **(****Figure 9B****).** In this analysis they found that in comparison to those organoids embedded in Matrigel, those embedded in cECM showed an overall upregulation of genes related to synaptic processes in overall. Among them, they found that the glutamatergic neuron marker vGLUT2 was upregulated in organoids embedded in cECM, showing a higher presence of glutamatergic neurons within them. In addition, genes related to calcium and potassium channels, such as CACBN2 and KCNK1, respectively, also showed a higher expression in these organoids. Finally, a group of genes related to the regulation and support of synaptic activity, such as OMG, NXPH1, GRM8, SYT4, NEFL and GABRA1, were found highly expressed in cECM organoids. Overall, the outcome of these analyses suggests that embedding organoids in cECM has a positive effect in neuronal maturation within the organoids.

### Example 2.3. Brain organoids can be successfully generated from amyotrophic lateral sclerosis (ALS) patients

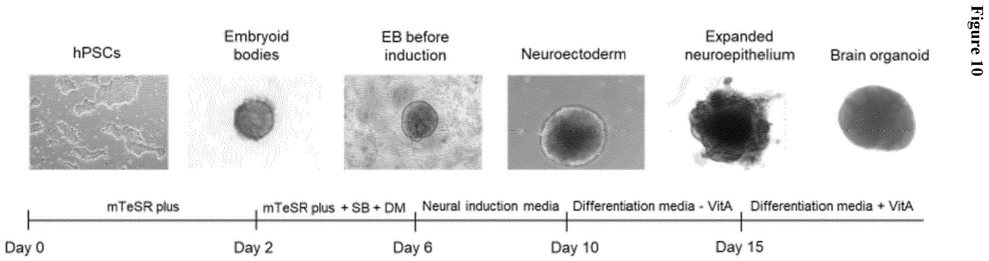

Once observed the abovementioned results, the inventors used human biological material, i.e., hiPSCs and cECM, from ALS patients and healthy control individual to generate a CBO model to study the disease. As previously demonstrated, protocol A organoids showed a better representation of cortical brain region, so they established this protocol as optimal in their case for the following experiments. Furthermore, they included the addition of porcine cECM to the protocol since they demonstrated before that it has a positive effect in the generation of CBOs **(****Figure 10****).**

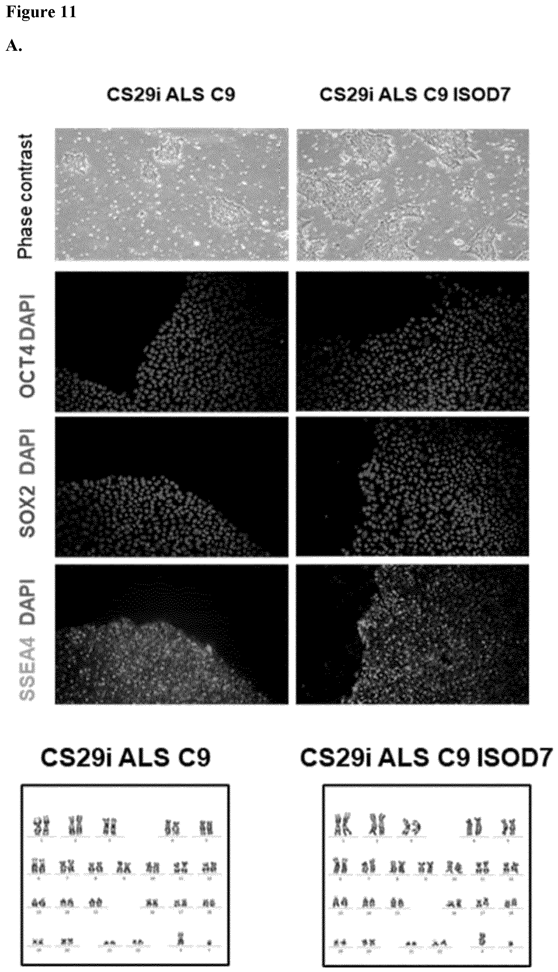

They performed two independent experiments with these samples. First, they assessed the generation of CBOs from two hiPSCs lines from an ALS patient: the original line obtained from the patient with a C9orf72 mutation (CS29iALS-C9n1), and the genetically corrected line obtained from that same patient (CS29iALS-C9n1 ISOD7) which does not contain the mutation. Before using these hiPSCs lines, pluripotency and normal karyotype were analyzed performing the same analyses as for the characterization of the line PRPF Control **(****Figure 11A****).** They also included the hiPSCs line PRPF control as a positive control. All hiPSCs lines successfully generated brain-like organoids. Organoids were observed under phase contrast microscopy. Organoids generated from hiPSCs derived from the ALS patient showed a similar structure as those generated from the positive control hiPSCs line PRPF control **(****Figure 11B****).**

After 2 months of maturation, ALS organoids display alterations in neurons such as degradation of neurofilaments (NF), detected by low intracellular expression of NF proteins **(****Figure 12B****).** NF levels increase in biological fluids (blood, cerebrospinal fluid) of ALS patients probably due to axon damage and NF degradation and measurement of NF levels is routinely used as a diagnosis and prognostic test in clinics. Furthermore, ALS organoids show a deregulation of pathways previously related to ALS physiopathology such as the ubiquitin-proteasome pathway **(****Figure 12C-G** ). Deregulation of the ubiquitin-proteasome pathway is mainly observed at the level of de-ubiquitinating enzymes, especially ubiquitin-specific proteases (USP) such us USP17 family, that is strongly downregulated **(****Figure 12D-E****)**.

On the other hand, they assessed the generation of cECM from brain samples of ALS patients and healthy control individuals. They continued following the procedure and conditions as the previously described study using porcine cECM. In this case, they decellularized 8 human brain samples for obtaining the cECM.

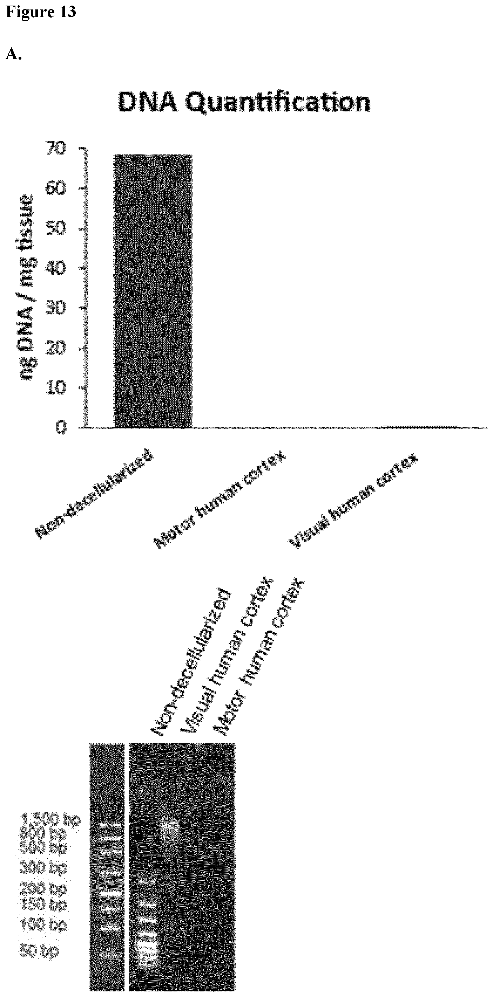

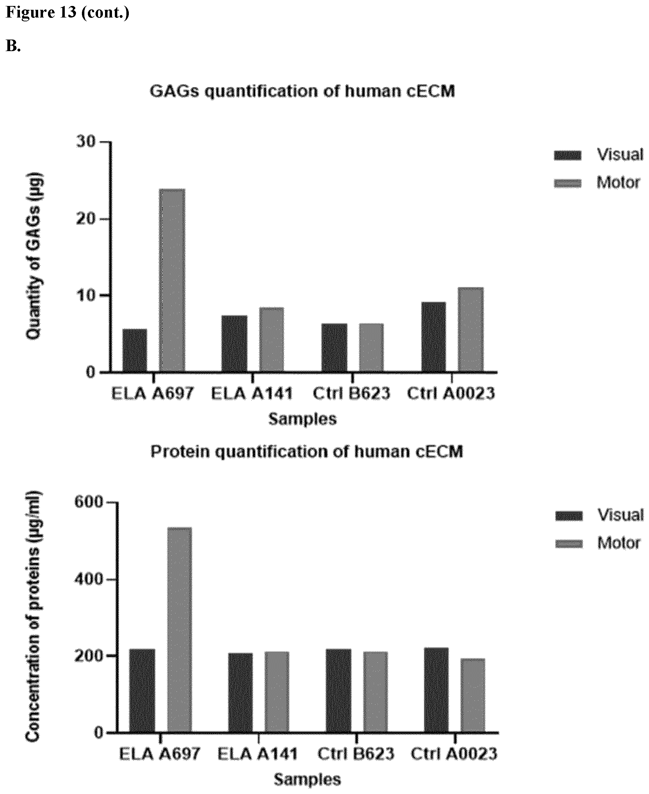

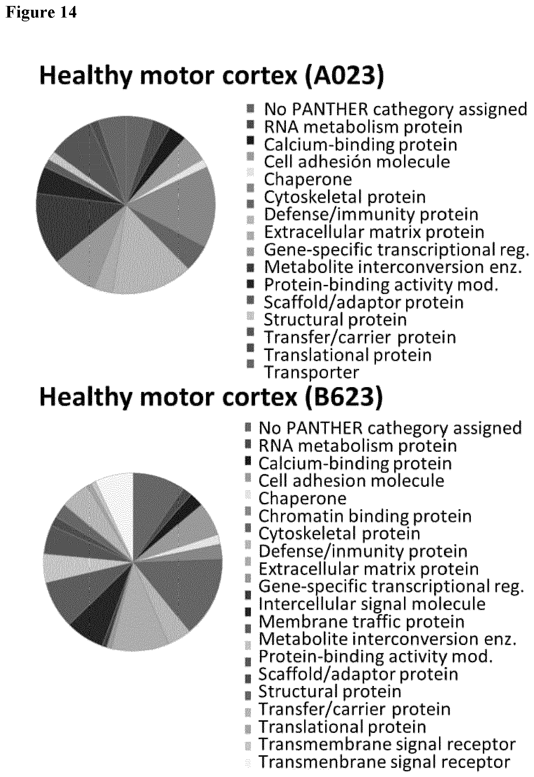

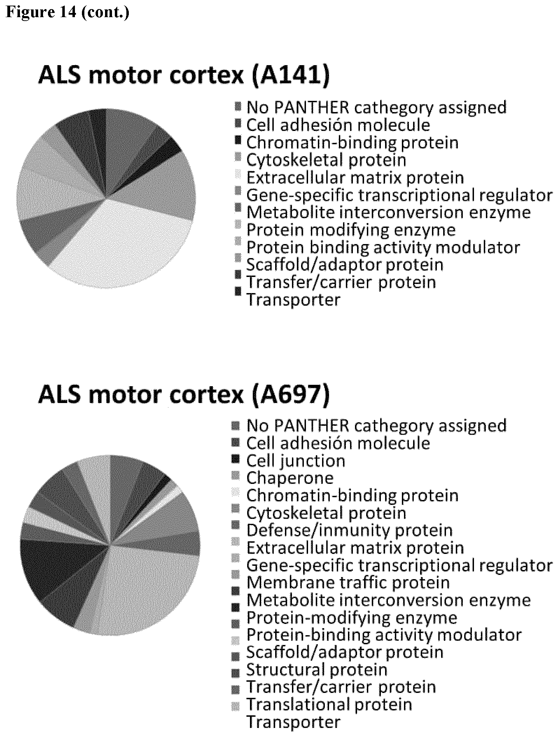

For confirming the successful decellularization of the samples, they performed several analyses. First, for verifying the removal of DNA, they performed a DNA extraction for quantification and analysis in an electrophoresis gel **(****Figure 13A****).** The DNA was removed successfully, showing no significant presence in the decellularized samples. Then, for verifying the preservation of essential compounds of the cECM, they performed a quantification of proteins and GAGs **(****Figure 13B****).** The protein quantification showed an equal preservation of proteins within all the samples but the motor cortex ECM of the ALS patient A697, which showed a higher concentration of proteins. The GAGs quantification was also equal in all samples but the motor cortex ECM of the same patient, which showed again a higher concentration. The higher concentration of GAGs and proteins in this sample might be due to the fact that this particular patient had fibrosis (an accumulation of ECM proteins). Proteomic profiling analysis showed differences in cECM obtained from ALS patients and healthy donors. Specifically, cECM from ALS patients showed less protein classes than cECM from healthy donors **(****Figure 14****).**

The inventors then generated CBOs from the hiPSC line PRPF control (derived from a healthy individual) by using the protocol A and the cECM from motor cortex of ALS patients. Organoids generated from the same hiPSC line but embedded in cECM from healthy individuals was used as control. No major differences were observed under phase contrast microscopy across groups **(****Figure 15A****).** However, transcriptome analysis showed that CBOs embedded in cECM from ALS patients showed deregulation of several signaling pathways **(****Figure 15B-C****)**. Interestingly, several ubiquitin peptidases (USP) were downregulated in ALS cECM embedded organoids.

### Example 2.4. Chemically induced aging of cortical organoids from healthy donors generated with cECM shows deregulation of the ubiquitin-proteasome pathway.

The inventors also used protocol A and the addition of porcine cECM to generate CBOs from hiPSC PRPF control line (derived from healthy donor) and artificially induce senescence in the organoids. This was done to study how aging alters biological processes at the cerebral cortex and compare these events with ALS pathological processes. Aging was chemically induced by treatment with SBI-0206965 (Zinc metalloprotease inhibitor) and Lopinavir (autophagy inhibitor) as described in **Figure 16A****.** Transcriptome analysis showed that aged organoids showed deregulation of several genes and signaling pathways, including USPs and proteasome-ubiquitin pathway **(****Figure 16B-C****)**. These results suggest that the proteasome-ubiquitin pathway could be an interesting target for treating ALS and age-related processes.

## Claims

1. An *in vitro* method for obtaining a cortical brain organoid, which comprises:
a) culturing one or more pluripotent stem cells (PSCs), in a maintenance medium for PSCs,
b) culturing the cells resulting from step a) in a neural induction medium, and
c) culturing the embryonic bodies resulting from step b) in contact with an extracellular matrix in the presence of a cortical differentiation medium comprising B27 supplement and N2 supplement, wherein the extracellular matrix is **characterized in that** it is a decellularized brain cortical extracellular matrix.

2. The *in vitro* method, according to the previous claim, wherein the maintenance medium for PSCs of a) comprises at least 90 ng/ml FGF-2, a ROCK inhibitor, and one or more inhibitors of the SMAD pathway.

3. The *in vitro* method, according to the previous claim, wherein the ROCK inhibitor is selected from the list consisting of: Y-27632, Fasudil (HA-1077), Ripasudil (K-115), H-1152, GSK269962A, SR3677, Thiazovivin, RKI-1447, Chroman 1 and Blebbistatin, preferably wherein the ROCK inhibitor is Y-27632.

4. The *in vitro* method, according to any of the previous claims, wherein the neural induction medium of b) comprises N2 supplement and/or one or more inhibitors of the SMAD pathway, wherein the N2 supplement is preferably at a concentration of at least 1%.

5. The *in vitro* method, according to any of the claims 2 to 4, wherein the one or more inhibitors of the SMAD pathway are selected from the list consisting of: SB431542, LY2157299 (Galunisertib), A-83-01, SD-208, RepSox (E-616452), LY2109761, SB525334, LY364947, Pirfenidone, SB505124, GW788388, Vactosertib (TEW-7197), ITD-1, Halofuginone, Sulfasalazine, LY 3200882, TP0427736 HCl, TGFβRI-IN-3, PD 169316, 3,3-Dimethyl-1-butanol, Lycopus Extract, AUDA, BIBF-0775, Ginsenoside Rh4, R-268712 and/or TA-02, Dorsomorphin, Dorsomorphin 2HCl, LDN-193189, LDN-193189 HCl, DMH1, K02288, LDN-212854, MI,347, LDN-214117, SIS3 and/or SIS3 HCl.

6. The *in vitro* method, according to the previous claim, wherein the one or more inhibitors of the SMAD pathway are SB431542 and Dorsomorphin.

7. The *in vitro* method for obtaining a cortical brain organoid of claim 1, according to any of the claims 1 to 6, wherein:
i) the maintenance medium for PSCs of a) comprises at least 90 ng/ml FGF-2, a ROCK inhibitor, and one or more inhibitors of the SMAD pathway,
ii) wherein the ROCK inhibitor is Y-27632,
iii) the neural induction medium of b) comprises N2 supplement at a concentration of at least 1% and one or more inhibitors of the SMAD pathway, and
iv) wherein the one or more inhibitors of the SMAD pathway are SB431542 and Dorsomorphin.

8. The *in vitro* method, according to any of the previous claims, wherein the one or more pluripotent stem cells (PSCs) are selected from: embryonic stem cells (ESCs) derived from a blastocyst stage embryo, induced pluripotent stem cells (iPSCs), parthenogenetic PSCs and PSCs generated by nuclear transfer.

9. A cortical brain organoid obtained or obtainable by the method of any of the claims 1 to 8.

10. The cortical brain organoid, according to claim 9, for use as a medicament.

11. The cortical brain organoid for use, according to claim 10, in a method of treating a brain disorder, condition, or disease.

12. Use of the cortical brain organoid of claim 9 for screening candidate compounds suitable for use in a method of treating a brain disorder, condition, or disease.

13. The cortical brain organoid for use, according to any of the claims 10 or 11, or use of the cortical brain organoid, according to claim 12, wherein the brain disorder, condition, or disease is a brain disorder, condition or disease that affects the cortical region of the brain.

14. The cortical brain organoid for use, according to the previous claim, or use of the cortical brain organoid, according to the previous claim, wherein the brain disorder, condition, or disease is selected from the list consisting of: amyotrophic lateral sclerosis (ALS), Alzheimer's disease, Parkinson's disease, epilepsy, dementia, encephalitis, cerebral tumor, cerebrovascular accident, Huntington's disease, Creutzfeldt-Jakob disease, Wilson's disease, Rasmussen's encephalitis, aphasia, frontotemporal dementia (FTD), cortical dysplasia, stroke, cerebral palsy, progressive supranuclear palsy, Lewy body dementia (LBD), cortical visual impairment (CVI), schizophrenia, autism spectrum disorder (ASD) and posterior cortical atrophy (PCA).

15. The cortical brain organoid for use, according to the previous claim, or use of the cortical brain organoid, according to the previous claim, wherein the brain disorder, condition, or disease is amyotrophic lateral sclerosis (ALS).
